# EUROPÄISCHE PATENTSCHRIFT

(11) **EP 1 105 403 B1**
(45) Veröffentlichungstag und Bekanntmachung des Hinweises auf die Patenterteilung: **04.04.2012**
(21) Anmeldenummer: 99945989.4
(22) Anmeldetag: 18.08.1999
(51) Int. Cl.: C07H 19/04, C07H 21/00, A61K 31/70, C12Q 1/68

(54) **3'-DESOXYPENTOPYRANOSYL-NUCLEINSÄURE UND IHRE HERSTELLUNG**
3'-DESOXYPENTOPYRANOSYL NUCLEIC ACID AND ITS PRODUCTION
ACIDE 3'-DESOXYPENTOPYRANOSYL-NUCLEIQUE ET SA PRODUCTION

(30) Priorität: 18.08.1998 DE 19837387
(43) Veröffentlichungstag der Anmeldung: 13.06.2001
(73) Patentinhaber: SANOFI, 75013 Paris (FR)
(72) Erfinder: MICULKA, Christian, A-1190 Wien (AT); WAGNER, Thomas, D-65719 Hofheim (DE); WINDHAB, Norbert, D-65795 Hattersheim (DE)
(74) Vertreter: Ackermann, Joachim
(86) Internationale Anmeldenummer: PCT/EP1999/006036
(87) Internationale Veröffentlichungsnummer: WO 2000/011011

(56) Entgegenhaltungen:
- WO-A-96/40711
- WO-A-98/25943
- B.DOBOSZEWSKI ET AL.: "Easy Synthesis and Different Conformational Behaviour of Purine and Pyrimidine B-D-Glycero-pent-2'-enopyranosyl Nucleosides." JOURNAL OF ORGANIC CHEMISTRY., Bd. 60, Nr. 24, 1995, Seiten 7909-7919, XP002122435 AMERICAN CHEMICAL SOCIETY. EASTON., US ISSN: 0022-3263
- DIEKMANN E ET AL: "DIDESOXY-RIBONUCLEOSIDE DURCH SCHMELZKONDENSATION DIDEOXY RIBONUCLEOSIDES BY FUSION METHOD" JOURNAL FUR PRAKTISCHE CHEMIE, CHEMIKER ZEITUNG,DE,WILEY VCH, WEINHEIM, Bd. 335, Nr. 5, Seite 415-424 XP000569335 ISSN: 1436-9966
- N.B.KHRIPACH ET AL.: "Glycosylation of N4-Benzoylcytosine and N6-Benzoyladenine by Acetals Glycals." KHIM. GETEROTSIKL. SOEDIN, Nr. 1, 1982, Seiten 111-117, XP002122436
- K.A.WATANABE ET AL.: "Nucleosides. 118. Total Syntheses of Pentopyranine B and D." CANADIAN JOURNAL OF CHEMISTRY, Bd. 59, Nr. 2, 1981, Seiten 468-472, XP002122437
- K.A.WATANABE ET AL.: "Nucleosides. LXXXVII. Total Syntheses of Pentopyranine A an a-L-Cytosine Nucleoside Elaborated by Streptomyces Griseochromogenes." JOURNAL OF ORGANIC CHEMISTRY., Bd. 39, Nr. 17, 1974, Seiten 2482-2486, XP002122438 AMERICAN CHEMICAL SOCIETY. EASTON., US ISSN: 0022-3263
- H.SETO ET AL.: "Biosynthesis of Blasticidin S. IV. Structures of Pentopyranines A and C, Two Cytosine Nucleosides with alpha-L-configuration." AGR. BIOL. CHEM., Bd. 37, Nr. 10, 1973, Seiten 2421-2426, XP002122439
- M BÖHRINGER ET AL: "110. Warum Pentose- und nicht Hexose-Nucleinsäuren ? Oligonucleotide aus 2',3'-Dideoxy-B-D-glucopyranosyl-Bausteine n ('Homo-DNS?): Herstellung" HELVETICA CHIMICA ACTA,CH,VERLAG HELVETICA CHIMICA ACTA. BASEL, Bd. 75, Nr. 5, Seite 1416-1477 XP002096856 ISSN: 0018-019X
- I SCHLÖNVOGT ET AL: "188. Pyranosyl-RNA('p-RNA'): NMR and Molecular Dynamics Study of the Duplex Formed by Self-Pairing of Ribopyranosyl-(C-G-A-A-T-T-C-G)" HELVETICA CHIMICA ACTA,CH,VERLAG HELVETICA CHIMICA ACTA. BASEL, Bd. 79, Nr. 8, Seite 2316-2346 XP002096829 ISSN: 0018-019X
- M BOLLI ET AL: "131. Pyranosyl-RNA: Further Observations on Replication" HELVETICA CHIMICA ACTA,CH,VERLAG HELVETICA CHIMICA ACTA. BASEL, Bd. 80, Nr. 6, Seite 1901-1951 XP002096832 ISSN: 0018-019X
- B DOBOSZEWSKI ET AL: "Synthesis of 3'-Deoxy-3'-C-Hydroxymethyl-aldopentopyran osyl Nucleosides and their Incorporation into Oligonucleotides. Part II" TETRAHEDRON,NL,ELSEVIER SCIENCE PUBLISHERS, AMSTERDAM, Bd. 51, Nr. 45, Seite 12319-12336-12336 XP002096831 ISSN: 0040-4020
- S PITSCH ET AL: "122. Pyranosyl-RNA ('p-RNA'): Base-Pairing Selectivity and Potential to Replicate" HELVETICA CHIMICA ACTA,CH,VERLAG HELVETICA CHIMICA ACTA. BASEL, Bd. 78, Nr. 7, Seite 1621-1635 XP002096830 ISSN: 0018-019X in der Anmeldung erwähnt
- ESCHENMOSER ET AL: "147. Why pentose- and not hexose-nucleic acids?" HELVETICA CHIMICA ACTA,CH,VERLAG HELVETICA CHIMICA ACTA. BASEL, Bd. 76, Seite 2161-2183 XP002094190 ISSN: 0018-019X in der Anmeldung erwähnt

## Beschreibung

Die vorliegende Erfindung betrifft 3'-Desoxypentopyranosyl-Nucleoside der Formel (I) oder der Formel (II), 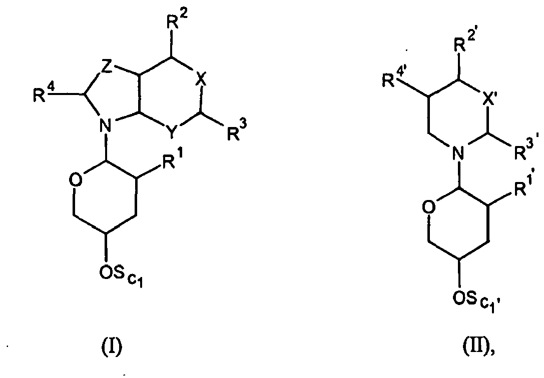 ihre Herstellung und ihre Verwendung zur Herstellung von 3'-Desoxypentopyranosyl-Nucleinsäuren.

Pyranosyl-Nucleinsäuren (p-NA's) sind im allgemeinen zur natürlichen RNA isomere Strukturtypen, bei denen die Pentose-Einheiten in der Pyranoseform vorliegen und durch Phosphodiestergruppen zwischen den Positionen C-2' und C-4' repetitiv verknüpft sind. Unter "Nucleobase" werden dabei die kanonischen Nucleobasen A, T, U, C, G, aber auch die Paare Isoguanin/Isocytosin und 2,6-Diaminopurin/Xanthin und im Sinne der vorliegenden Erfindung auch andere Purine und Pyrimidine verstanden. P-NA's, und zwar die von der Ribose abgeleitete p-RNA's, wurden zum erstenmal von Eschenmoser et al. beschrieben (Hel. Chim. Acta 1993, 76, 2126; Helv. Chim. Acta 1995, 78, 1621; Angew. Chem. 1996, 108, 1619-1623; WO 98/25943, Helv. Chim. Acta, 1996, 79, 2316, Helv. Chim. Acta, 1997, 80, 1901). Sie bilden ausschließlich sogenannte Watson-Crick-gepaarte, d. h. Purin-Pyrimidin-und Purin-Purin-gepaarte, antiparallele, reversibel "schmelzende", quasi-lineare und stabile Duplices. Homochirale p-RNA-Stränge entgegengesetzten Chiralitätssinns paaren ebenfalls kontrollierbar und sind in der gebildeten Duplex streng nicht-helical. Diese für den Aufbau supramolekularer Einheiten wertvolle Spezifität hängt mit der relativ geringen Flexibilität des Ribopyranosephosphat-Rückgrats sowie mit der starken Neigung der Basenebene zur Strangachse und der hieraus folgenden Tendenz zu intercatenarer Basenstapelung im resultierenden Duplex zusammen und läßt sich letzlich auf die Teilnahme eines 2',4'-cis-disubstituierten Ribopyranoserings am Aufbau des Rückgrates zurückführen. Diese wesentlich besseren Paarungseigenschaften machen p-NA's gegenüber DNA und RNA für die Anwendung des Aufbaus supramolekularer Einheiten zu bevorzugten Paarungssystemen. Sie bilden ein zu natürlichen Nucleinsäuren orthogonales Paarungsystem, d. h. sie paaren nicht mit in der natürlichen Form vorkommenden DNA's und RNA's, was im besonderen im diagnostischen Bereich von Bedeutung ist.

Die p-RNA zeigt jedoch folgende Nachteile, die auf die Anwesenheit der 3'-Hydroxylfunktion zurückzuführen sind:
1. Der notwendige Schutz der 3'-Hydroxylgruppe mit einer Benzoylschutzgruppe erschwert und verlängert den Syntheseweg zu den monomeren Bausteinen erheblich.
2. Aufgrund der Verwendung des Allylrestes als Basen- und Phosphatschutzgruppe muß die Entschützung und die Abspaltung des Oligonucleotids durch zwei hintereinandergeschaltete Schritte erfolgen. Zuerst werden die Allylreste nach der Noyori-Methode (R. Noyori, J. Am. Chem. Soc. 1990, 112, 1691-6) entfernt. Anschliessend müssen die basenlabilen Acylgruppen abgespalten und das Oligonucleotid vom Träger entfernt werden.
3. Nach beendeter Oligonucleotidsynthese bereitet die Abspaltung der 3'-Benzoylreste vom Oligonucleotid Schwierigkeiten. Um diese Reste effektiv zu entfernen ist die Verwendung von Hydrazin notwendig, was zu Ringöffnungen der Pyrimidinbasen, vor allem Uracil und Thymin, führen kann.
4. Bei der Synthese der Oligonucleotide wird 5-(4-Nitrophenyl)-1H-tetrazol als Kupplungsreagenz in der automatisierten p-RNA-Synthese eingesetzt. Die Konzentration dieses Reagenz in der Lösung von Tetrazol in Acetonitril ist dabei so hoch, daß im allgemeinen das 5-(4-Nitrophenyl)-1H-tetrazol in den dünnen Schläuchen des Synthesizers Auskristallisiert und die Synthese somit zu einem vorzeitigen Ende kommt. Zudem wurde beobachtet, daß die Oligomeren mit 5-(4-Nitrophenyl)-1H-tetrazol verunreinigt waren. Auch das alternativ verwendete Benzimidazolium-Triflat weist negative Seiten auf: es kristallisiert, wenn auch seltener, in den Schläuchen aus, ist teuer und muß vor seiner Verwendung zudem umkristallisiert werden.

In Doboszweski B. et al., J. Org. Chem., 1995, Vol. 60, S. 7909; Dieckmann E. et al. J. Prak. Chem, 1993, Vol. 335, S. 415, Kripach N.B. et al. Khim. Geterotskil. Soedin, 1982, S. 111; Watanabe K.A. et al., Can. J. Chem., 1981, S. 468; Watanabe K.A. et al., Am. Chem. Soc., 1974, S. 2482; Seto H. et al., Biosynthesis of Blasticidin, 1973, S. 2421; Böhringer M. et al., Hel. Chim. Act., 1992, Vol. 75, S. 1416; Doboszewski et al., Tetrahedron, 1995, Vol. 51, S. 12319, WO 96/40711 sind spezielle Pent-2'-enopyranosyl-, Didesoxyribon-nucleoside, glycosilierte Nucleoside bzw. Pentopyranine beschrieben.

Aufgabe der vorliegenden Erfindung war es daher, neue Pentopyranosyl-Nucleoside für orthogonale Paarungssysteme bereitzustellen und zu oligomerisieren, wodurch die oben beschriebenen Nachteile umgangen werden können.

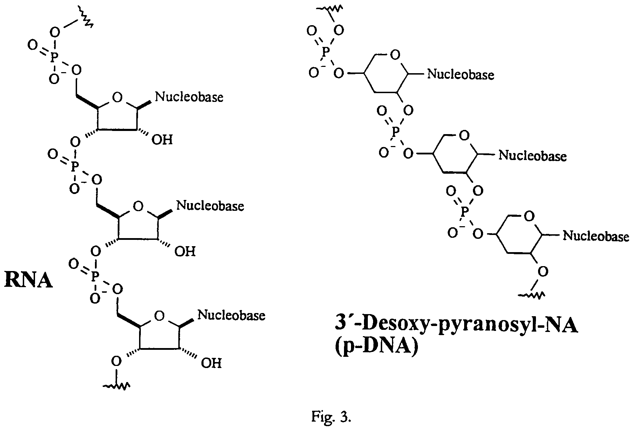

Überraschenderweise wurde nun gefunden, daß 3'-Desoxypentopyranosyl-Nucleinsäuren (p-DANN) die beschriebenen Nachteile nicht aufweisen und dennoch die vorteilhaften orthogonalen Paarungseigenschaften besitzen (siehe Fig. 3).

Ein Gegenstand der vorliegenden Erfindung sind daher 3'-Desoxypentopyranosyl-Nucleoside der Formel (I), 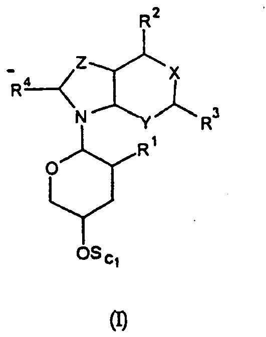 worin
R¹ gleich , OH, Hal mit Hal gleich Br oder Cl, ein Rest ausgewählt aus 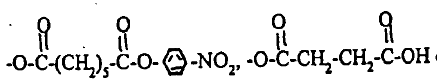 oder -O-P(N(i-Pr)₂] (OCH₂CH₂CN) mit i-Pr gleich Isopropyl, oder -O-PH-(=O)(-O-),
R², R³ und R⁴ unabhängig voneinander, gleich oder verschieden, jeweils H, NR⁵R⁶, OR⁷, SR⁸, =O, CₙH₂ₙ₊₁ mit n eine ganze Zahl von 1-12, vorzugsweise 1-8, insbesondere 1-4, eine β-eliminierbare Gruppe der Formel-OCH₂CH₂R¹⁸ mit R¹⁸ gleich ein
Cyano- oder p-Nitrophenylrest oder ein Fluorenylmethyloxycarbonyl-(Fmoc)-Rest, oder (CₙH₂ₙ)NR¹⁰R¹¹ mit R¹⁰R¹¹ gleich H, CₙH₂ₙ₊₁ oder R¹⁰R¹¹ verbunden über einen Rest der Formel 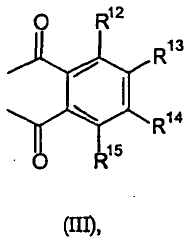 worin R¹², R¹³, R¹⁴ und R¹⁵ unabhängig voneinander, gleich oder verschieden, jeweils H, OR⁷, oder CₙH₂ₙ₊₁, oder CₙH₂ₙ₋₁, wobei n die
oben genannte Bedeutung hat, bedeuten und
R⁵, R⁶, R⁷ und R⁸ unabhängig voneinander, gleich oder verschieden, jeweils H, CₙH₂ₙ₊₁, oder CₙH₂ₙ₋₁, wobei n die oben genannte Bedeutung hat, -C(O)R⁹ mit R⁹ gleich ein linearer oder verzweigter, gegebenenfalls substituierter Alkyl-, Aryl-, vorzugsweise Phenyl-Rest,
X, Y und Z unabhängig voneinander, gleich oder verschieden, jeweils =N-, =C(R¹⁶)- oder - N(R¹⁷)- mit R¹⁶ und R¹⁷ unabhängig voneinander, gleich oder verschieden, jeweils H oder CₙH₂ₙ₊₁ oder (CₙH₂ₙ)NR¹⁰R¹¹ mit den oben genannten Bedeutungen, bedeutet, und
S_{cl} Wasserstoff oder eine Schutzgruppe ausgewählt aus einer Acyl-, Trityl-, Allyloxycarbonyl-, einer photolabilen Schutzgruppe, vorzugsweise eine Benzoyl-, Fluorenylmethyloxycarbonyl-(Fmoc)- oder 4, 4'-Dimethoxytrityl-(DMT)-gruppe, ist,
oder der Formel (II) 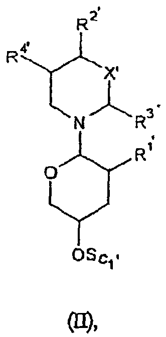 worin R^{1'} gleich OH, Hal mit Hal gleich Br oder Cl, ein Rest ausgewählt aus 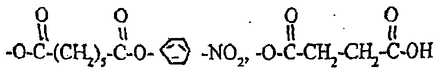 oder -O-P[N(i-Pr)₂] (OCH₂CH₂CN) mit i-Pr gleich Isopropyl, oder -O-PH-(=O)(-O-)
R^{2'},R^{3'} und R^{4'} unabhängig voneinander, gleich oder verschieden, jeweils =O, CₙH₂ₙ₊₁ oder OCₙH₂ₙ₋₁, eine β-eliminierbare Gruppe der Formel -OCH₂CH₂R¹⁸
mit R¹⁸ gleich ein Cyano- oder p-Nitrophenyhest oder ein fluorenylmethyloxycarbonyl-(Fmoc)-Rest oder (CₙH₂ₙNR^{10'}R^{11'}, wobei R^{10'}, R^{11'}, unabhängig voneinander die oben genannte Bedeutung von R¹⁰ bzw. R¹¹ hat, und
X' =N-, =C(R^{16'})- oder -N(R^{17'})- bedeutet, R^{16'} und R^{17'} unabhängig voneinander die oben genannte Bedeutung von R¹⁶ und R¹⁷ haben, und S_{cl}, die oben genannte Bedeutung von S_{cl} hat.

Die 3'-Desoxypentopyranosyl-Nucleoside sind im allgemeinen 3'-Desoxyribo-, 3'-Desoxyarabino-, 3'-Desoxylyxo-und/oder 3'-Desoxyxylo-pyranosyl-Nucleoside,
vorzugsweise 3'-Desoxyribopyranosyl-Nucleoside, wobei der 3'-Desoxypentopyranosyl-Teil D-konfiguriert, aber auch L-konfiguriert sein kann.

Üblicherweise handelt es sich bei den 3'-Desoxypentopyranosyl-Nucleosiden um 3'-Desoxypentopyranosyl-purin, -2,6-diaminopurin, -6-purinthiol, -adenin, -guanim, -isoguanin; -xanthin, -hypoxanthin, -thymin, -indol, -tryptamin, -N-phthaloyltryptamin, -coffein, -theobromin, -theophyllin, insbesondere um 3'-Desoxypentopyranosyl-purin,--adenin, -guanin, -thymin, tryptamin oder -N-phthaloryptamin.

Unter die Verbindungen fallen auch 3'-Desoxypentopyranosyl-Nucleoside, die als Linker verwendet werden können, d. h. als Verbindungen mit funktionellen Gruppen, die kovalent an Biomoleküle, wie z. B. in ihrer natürlichen Form vorkommende oder modifizierte Nucleinsäuren, wie DNA, RNA aber auch p-NA's, vorzugsweise p-DNA's, binden können.

Beispielsweise fallen hierunter 3'-Desoxypentopyranosyl-Nucleoside, bei denen R², R³, R⁴, R^{2'}, R^{3'} und/oder R^{4'} ein 2-Phthalimidoethyl-Rest bedeutet.

Geeignet sind auch Uracil-basierende Linker, bei denen vorzugsweise die 5-Position des Uracils modifiziert wurde, z. B. N-Phthaloylaminoethyluracil, aber auch Indol-basierende Linker, vorzugsweise Tryptaminderivate, wie z. B. N-Phthaloyltryptamin.

Darüberhinaus werden von der vorliegenden Erfindung 3'-Desoxypentopyranosyl-Nueleoside umfaßt, die ausschließlich am 4'-Sauerstoffatom des 3'-Desoxypentopyranosid-Teils eine Schutzgruppe, insbesondere eine Tritylgruppe, besonders bevorzugt eine Dimethoxytritylgruppe, tragen.

Folgende Verbindungen stellen bevorzugte Beispiele von Pentopyranosyl-Nucleosiden dar, die in der erfindungsgemäßen Nucleinsäure vorhanden sein können, bzw. für deren Herstellung besonders geeignet sind:
A) [(2',4'-Di-O-Benzoyl)-3'-desoxy-β-ribopyranosyl]-Nucleoside, inbesondere ein [(2',4'-Di-O-Benzoyl)-3'-desoxy-β-ribopyranosyl]-adenin, -guanin, -thymidin, -xanthin oder -hypoxanthin, sowie ein N-Benzoyl-2',4'-di-O-benzoyl-3'-desoxy-ribopyranosyl-Nucleosid, insbesondere ein -adenin oder -guanin, sowie ein N-Isobutyroyl-2',4"di-O-benzoyl-3'-desoxy-ribopyranosyl-Nucleosid, insbesondere ein -adenin oder -guanin sowie ein O⁶-(2-Cyanoethyl)-N²-isobutyroyl-2',4'-di-O-benzoyl-3'-desoxyribopyranosyl-Nucleosid, insbesondere ein -guanin, sowie ein O⁶-(2-(4-Nitrophenyl)ethyl)-N²-isobutyroyl-2',4'-di-O-benzoyl-3'-desoxy-ribopyranosyl-Nucleosid, insbesondere ein - guanin.
B) 3'-Desoxy-β-ribopyranosyl-Nucleoside, inbesondere ein 3'-Desoxy-β-ribopyranosyladenin, -guanin, -thymidin, -xanthin oder hypoxanthin, sowie ein N-Benzoyl-, N-Isobutyroyl-, O⁶-(2-Cyanoethyl)- oder O⁶-(2-(4-Nitrophenyl)ethyl)-N²-isobutyroyl-3'-Desoxy-β-ribopyranosyl-Nucleosid, insbesondere ein -guanin.
C) 4'-DMT-3'-desoxy-pentopyranosyl-Nucleoside, vorzugsweise ein 4'-DMT-3'-desoxyribopyranosyl-Nueleosid, insbesondere ein 4'-DMT-3'-desoxyribopyranosyl-adenin, -guanin, -thymidin, -xanthin oder -hypoxanthin, sowie ein N-Benzoyl-4'-DMT-3'-desoxy-ribopyranosyl-Nucleosid, insbesondere ein N-Benzoyl-4'-DMT-3'-desoxy-ribopyranosyl-adenin oder -guanin, sowie ein N-Isobutyroyl-4'-DMT-3'-desoxyribopyranosyl-Nucleosid, insbesondere ein N-Isobutyroyl-4'-DMT-3'-desoxy-ribopyranosyl-adenin oder -guanin sowie ein O⁶-(2-Cyanoethyl)-N²-isobutyroyl-4'-DMT-3'-desoxyribopyranosyl-Nucleosid, insbesondere ein O⁶-(2-Cyanoethyl)-N²-isobutyroyl-4'-DMT-3'-desoxy-ribopyranosyl-guanin, sowie ein O⁶-(2-(4-Nitrophenyl)ethyl)-N²-isobutyroyl-4'-DMT-3'-desoxyribopyranosyl-Nucleosid, insbesondere ein O⁶-(2-(4-Nitrophenyl)ethyl)-N²-isobutyroyl-4'-DMT-3'-desoxyribopyranosyl-guanin.
D) 3'-Desoxy-β-ribopyranosyl-N,N'-dibenzoyl-adenosin oder 3'-Desoxy-β-Ribopyranosyl-N,N'-dibenzoyl-guanosin.

Als Vorstufe für die Oligonucleotidsynthese eignen sich beispielsweise 4'-DMT-3'-desoxypentopyranosyl-Nucleoside-2'-phosphitamid/-H-phosphonat, vorzugsweise ein 4'-DMT-3'-desoxy-ribopyranosyl-Nucleosid-2'-phosphitamid/-H-phosphonat, insbesondere ein 4'-DMT-3'-desoxyribopyranosyl-adenin- , -guanin- , -thymidin-, -xanthin,- oder -hypoxanthin 2'-phosphitamid/-H-phosphonat, sowie ein N-Benzoyl-4'-DMT-3'-desoxy-ribopyranosyl-adenin- oder -guanin 2'-phosphitamid/-H-phosphonat sowie ein N-Isobutyroyl-4'-DMT-3'-desoxy-ribopyranosyl-adenin-, oder -guanin 2'-phosphitamid/-H-phosphonat, O⁶-(2-Cyanoethyl)-4'-DMT-3'-desoxy-ribopyranosyl-guanin-, -xanthin-,-hypoxanthin-2'-phosphitamid/-H-phosphonat oder O⁶-(2-(4-Nitrophenyl)ethyl)-N²-isobutyroyl-4'-DMT-3'-desoxyribopyranosyl-guanin, und für die Kopplung an den festen Träger beispielsweise 4'-DMT-3'-desoxy-pentopyranosyl-Nucleoside-2'-succinat, vorzugsweise ein 4'-DMT-3'-desoxy-ribopyranosyl-Nucleosid-2'-succinat, insbesondere ein 4'-DMT-3'-desoxyribopyranosyl-adenin- , -guanin- , -thymidin-, -xanthin-, oder-hypoxanthin -2'- succinat sowie ein N-Benzoyl-4'-DMT-3'-desoxyribopyranosyl-adenin-, oder -guanin oder -2'-succinat sowie ein N-Isobutyroyl-4'-DMT-3'-desoxy-ribopyranosyl-adenin-, oder -guanin -2'- succinat, O-(2-Cyanoethyl)-4'-DMT-3'-desoxy-ribopyranosyl-guanin -2'-succinat sowie ein O⁶-(2-(4-Nitrophenyl)ethyl)-N²-isobutyroyl-4'-DMT-3'-desoxyribopyranosyl-guanin-2'-succinat.

Die 3'-Desoxyribopyranosyl-nucleoside können z.B. durch ein Verfahren hergestellt werden, bei dem
(a) eine gegebenenfalls geschützte Nucleobase mit einer geschützten 3'-Desoxyribopyranose umgesetzt wird,
(b) die Schutzgruppen von dem 3'-Desoxyribopyranosyl-Teil des Produktes aus Schritt (a) abgespalten werden, und gegebenenfalls
(c) das Produkt aus Schritt (b) an der 4'-Position des 3'-Desoxypentopyranosid geschützt wird.

In einer besonderen Ausführungsform ist das 3'-Desoxy-pyranosylnucleosid durch eine säurelabile, basenlabile, photolabile β-eliminierbare oder metallkatalysiert abspaltbare Schutzgruppe S_{cl}, oder S_{cl}, geschützt.

Im allgemeinen handelt es sich bei den genannten Schutzgruppen um eine ß-eliminierbare Schutzgruppe, vorzugsweise eine Fluorenylmethyloxycarbonyl-(Fmoc) Gruppe, um eine photolabile Schutzgruppe, um eine Acylgruppe, vorzugsweise um eine Acetyl-, Benzoyl-, Nitrobenzoyl- und/oder Methoxybenzoyl-Gruppe, oder um Tritylgruppen, vorzugsweise um eine 4, 4'-Dimethoxytrityl-(DMT)-Gruppe.

So erfolgt beispielsweise die Einführung einer DMT-Gruppe durch Umsetzen mit DMTCl in Anwesenheit einer Base, z. B. von N-Ethyldiisopropylamin (Hünig-Base), und z. B. von Pyridin, Methylenchlorid oder einem Pyridin/Methylenchlorid-Gemisch bei Raumtemperatur.

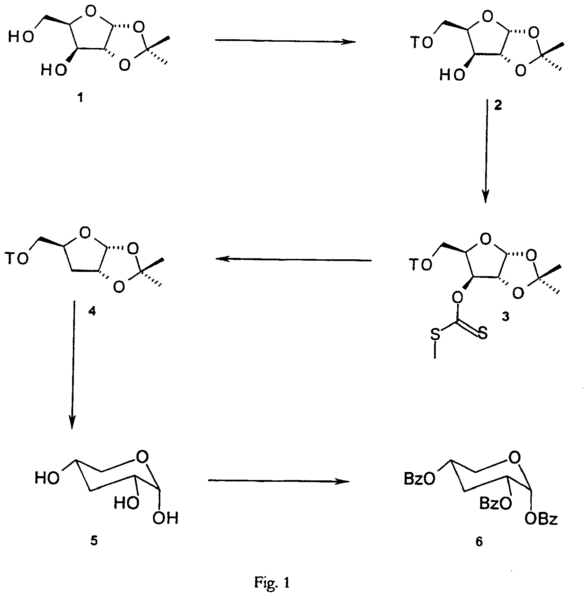

Der vorzugsweise anomerenreine Ribopyranosyl-Baustein wird im allgemeinen ausgehend von der 1,2-O-Isopropyliden-5-O-triphenylmethyl-α-D-xylofuranose (1 in Fig. 1) nach bekannten Verfahren (W. Sowa, Can. J. Chem, 1968, 46, 1568; Z.J. Witczak et. al. Carbohydrate Research, 1982, 110. 326) aber im allgemeinen mit verbesserten Ausbeuten hergestellt. Analog dem bekannten Verfahren wird 1 (Fig. 1) trityliert und vorzugsweise Natriumhydrid an Stelle von Natronlauge für die Darstellung des Methylxanthogenatesters in der 3'-Position (₂ in Fig. 1) verwendet. Nach Entfernen des Methylxanthogenats werden die Trityl- und die Isopropylidenschutzgruppe vorzugsweise mit Trifluoressigsäure an Stelle des in der Literatur beschriebenen 80% Eisessigs abgespalten. Durch diese Modifikationen konnten die Ausbeuten z.T. erheblich verbessert werden.

In einer weiteren Ausführungsform wird ein Linker gemäß Formel (II), worin R^{4'} (CₙH₂ₙ)NR^{10'}R^{11'} bedeutet und R^{10'}R^{11'} über einen Rest der Formel (III) mit der bereits bezeichneten Bedeutung verbunden ist, durch folgendes Verfahren auf vorteilhafte Weise hergestellt:
(a) eine Verbindung der Formel (II) mit R^{4'} gleich (CₙH₂ₙ)OS_{c3} oder (CₙH₂ₙ)Hal, worin n die oben genannte Bedeutung hat, S_{c3} eine Schutzgruppe, vorzugsweise eine Mesylat-Gruppe, und Hal Chlor oder Brom bedeutet, wird mit einem Azid, vorzugsweise in DMF, umgesetzt, anschließend wird
(b) das Reaktionprodukt aus (a), vorzugsweise mit Triphenylphosphin z. B. in Pyridin reduziert, dann
(c) das Reaktionsprodukt aus (b) mit einem entsprechenden Phthalimid, z. B. N-Ethoxycarbonylphthalimid, umgesetzt, und
(d) das Reaktionsprodukt aus (c) mit einer entsprechenden geschützten Pyranose, z. B. 2',4'-Di-O-Benzoyl-3'-desoxyribopyranose, umgesetzt, und schließlich
(e) werden die Schutzgruppen, z. B. mit Methylat, abgespalten, und
(f) die weiteren Schritte, wie oben bereits beschrieben, durchgeführt.

Daneben weisen Indolderivate als Linker den Vorzug der Fluoreszenzfähigkeit auf und sind daher für Nanotechnologie-Anwendungen, bei denen es ggf. um den Nachweis kleinster Substanzmengen geht, besonders bevorzugt. So wurden Indol-1-riboside bei N. N. Suvorov et al., Biol. Aktivn. Soedin., Akad. Nauk SSSR 1965, 60 und Tetrahedron 1967, 23, 4653 bereits beschrieben. Allerdings gibt es kein analoges Verfahren, 3-substituierte Derivate herzustellen. Im allgemeinen erfolgt ihre Herstellung über die Bildung eines Aminals der ungeschützten Zuckerkomponente und einem Indolin, welches dann durch Oxidation in das Indol-1-ribosid übergeführt wird. Beschrieben wurden z. B. Indol-1-glucoside und -1-arabinoside (Y. V. Dobriynin et al, Khim.-Farm. Zh. 1978, 12, 33), deren 3-substituierte Derivate meist über Vielsmeier-Reaktion hergestellt wurden. Dieser Weg der Einführung von Aminoethyl-Einheiten in 3-Position des Indols ist für eine industrielle Anwendung jedoch zu aufwendig.

In einer weiteren besonderen Ausführungsform wird daher ein Linker gemäß Formel (I), worin X und Y unabhängig voneinander, gleich oder verschieden, jeweils =C(R¹⁶) mit R¹⁶ gleich H oder CₙH₂ₙ und Z =C(R¹⁶)- mit R¹⁶ gleich (CₙH₂ₙ)NR¹⁰R¹¹ durch folgendes Verfahren auf vorteilhafte Weise hergestellt:
(a) das entsprechende Indolin, z. B. N-Phthaloyltryptamin, wird mit einer Pyranose, z. B. D-3'-Desoxyribose, zum Nucleosiddiol umgesetzt, dann werden
(b) die Hydroxylgruppen des Pyranosyl-Teils des Produktes aus (a) vorzugsweise mit Acylgruppen, z. B. mittels Essigsäureanhydrid, geschützt, anschließend wird
(c) das Produkt aus (b), z. B. durch 2,3-Dichlor-5,6-dicyanoparachinon, oxidiert, und
(d) die Hydroxyl-Schutzgruppen des Pyranosyl-Teils des Produktes aus (c) werden z. B. mittels Methylat abgespalten und anschließend werden
(e) die weiteren Schritte, wie oben bereits beschrieben, durchgeführt.

In einer weiteren Ausführungsform werden die 4'- bzw. 2'-geschützten, 3'-Desoxypentopyranosyl-Nucleoside in einem weiteren Schritt phosphityliert oder an eine feste Phase gebunden.

Die Phosphitylierung erfolgt beispielsweise durch Phosphorigsäurecyanoethylesterdiisopropyl-amidchlorid in Anwesenheit einer Base, z. B. N-Ethyldiisopropylamin oder durch Phosphortrichlorid und Imidazol bzw. Tetrazol und nachfolgender Hydrolyse unter Basenzusatz. Im ersten Fall ist das Produkt ein Phosphoramidit und im zweiten Fall ein H-Phosphonat. Die Bindung eines geschützten erfindungsgemäßen Pentopyranosyl-Nucleosids an eine feste Phase, z. B. "long-chain-alkylamino-controlled pore glass" (CPG, Sigma Chemie, München) kann beispielsweise über einen Succinoyl-Linker erfolgen.

Die erhaltenen Verbindungen können für die Herstellung der erfindungsgemäßen 3'-Desoxypentopyranosyl-Nucleinsäuren dienen.

Ein weiterer Gegenstand der vorliegenden Erfindung ist daher ein Verfahren zur Herstellung einer 3'-Desoxypentopyranosyl-Nucleinsäuregemäβ Anspruch 7 mit folgenden Schritten:
(a) in einem ersten Schritt wird ein geschütztes 3'-Desoxypentopyranosyl-Nucleosid, wie oben bereits beschrieben, an eine feste Phase gebunden und
(b) in einem zweiten Schritt wird das gemäß Schritt (a) an eine feste Phase gebundene 4'-geschützte 3'-Desoxypentopyranosylnukleosid um ein 2'-phosphityliertes 4'-geschütztes 3'-Desoxypentopyranosyl-Nucleosid verlängert und bei Einsatz von Phosphoramiditen anschließend z. B. durch eine wäßrige Jodlösung oxidiert und
(c) Schritt (b) solange mit gleichen oder unterschiedlichen phosphitylierten 3'-, 4'-geschützten 3'-Desoxypentopyranosyl -Nucleosiden wiederholt, bis die gewünschte 3'-Desoxypentopyranosyl -Nucleinsäure vorliegt.

Bei Einsatz von H-Phosphonaten erfolgt die Oxidation zu den entsprechenden Phosphorsäurediestern im allgemeinen am Ende der Reaktionskette z.B. durch eine wäßrige Jodlösung.

Als Kupplungsreagenz bei Einsatz von Phosphoramiditen eignet sich besonders Pyridinium-Hydrochlorid, da im Gegensatz zu üblicherweise verwendeten Kupplungsreagenzien keine Umkristallisation des Kupplungsreagenzes, keine Verstopfung der Kupplungsreagenz-Leitungen und eine wesentlich schnellere Kondensation erfolgt.

Als Kupplungsreagenz beim Einsatz von H-Phosphonaten eignen sich besonders Arylsulfonylchloride, Diphenylchlorophosphat, Pivaloylchlorid oder Adamantoylchlorid.

Ein wesentlicher Vorteil der H-Phosphonatmethode ist, daß keine Phosphatschutzgruppen benötigt werden. Die Acylschutzgruppen der Basen können z.B. durch wäßrigen Ammoniak abgespalten werden. Bei Verwendung des 2-(4-Nitrophenyl)ethyl-Restes als Schutzgruppe der O⁶-Position des Guanins kann dieser beispielsweise problemlos durch eine ca. 40minütige Behandlung mit 1M DBU entfernt werden.

Weiterhin ist es von Vorteil, daß keine schutzgruppenabspaltenden Hydrazinolyse von Oligonukleotiden notwendig ist, und somit keine Ringöffnung, vor allem bei Uracil und Thymin, zu befürchten ist. Die Cyanoethylreste können gemeinsam mit den Acylschutzgruppen der Basen durch wäßrigen Ammoniak abgespalten werden. Bei der Verwendung des 2-(4-Nitrophenyl)ethyl-Restes als Schutzgruppe der O⁶-Position des Guanins kann der Rest ohne Probleme durch ca. 40 minütige Behandlung mit 1M DBU entfernt werden.

In einer weiteren besonderen Ausführungsform können in Schritt (a) und/oder Schritt (b) auch Pentofuranosyl-nucleoside, z. B. das in ihrer natürlichen Form vorkommende Adenosin, Guanosin, Cytidin, Thymidin und/oder Uracil, eingebaut werden, was z. B. zu einer gemischten p-DNA-DNA bzw. p-DNA-RNA führt.

p-NA's und insbesondere die p-DNA's bilden untereinander stabile Duplices und paaren im allgemeinen nicht mit den in ihrer natürlichen Form vorkommenden DNA's und RNA's. Diese Eigenschaft macht p-NA's zu bevorzugten Paarungssystemen.

Solche Paarungssysteme sind supramolekulare Systeme nicht kovalenter Wechselwirkung, die sich durch Selektivität, Stabilität und Reversiblität auszeichnen, und deren Eigenschaften bevorzugt thermodynamisch, d. h. durch Temperatur, pH-Wert und Konzentration beeinflußt werden. Solche Paarungssysteme können z. B. aufgrund ihrer selektiven Eigenschaften auch als "molekularer Klebstoff" für die Zusammenführung von unterschiedlichen Metallelustern zu Cluster-Verbänden mit potentiell neuen Eigenschaften verwendet werden [siehe z. B. R. L. Letsinger, et al., Nature 1996, 382, 607-9; P. G. Schultz et al., Nature 1996, 382, 609-11]. Folglich eignen sich die p-NA's auch für die Anwendung im Bereich der Nanotechnologie, beispielsweise zur Herstellung neuer Materialien, Diagnostika und Therapeutika sowie mikroelektronischer, photonischer bzw. optoelektronischer Bauteile und für das kontrollierte Zusammenführen molekularer Species zu supramolekularen Einheiten, wie z. B. für den (kombinatorischen) Aufbau von Proteinassemblies [siehe z. B. A. Lombardi, J. W. Bryson, W. F. DeGrado, Biomoleküls (Pept. Sci.) 1997, 40, 495-504], da p-NA's Paarungssysteme bilden, die stark und thermodynamisch kontrollierbar sind. Eine weitere Anwendung ergibt sich daher gerade im diagnostischen und drug discovery-Bereich durch die Möglichkeit, funktionelle, bevorzugt biologische Einheiten wie Proteine oder DNA/RNA-Abschnitte, mit einem p-NA-Code zu versehen, der nicht mit den natürlichen Nucleinsäuren interferiert (siehe z. B. WO93/20242).

Ein Biomolekül, z. B. DNA oder RNA, kann zum nicht-kovalenten Verbinden (Linken) mit einem anderen Biomolekül, z. B. DNA oder RNA, verwendet werden, wenn beide Biomoleküle Abschnitte enthalten, die aufgrund komplementärer Sequenzen von Nucleobasen durch Ausbildung von Wasserstoffbrücken aneinander binden können. Derartige Biomoleküle finden z. B. in analytischen Systemen zur Signalamplifizierung Verwendung, wo ein in seiner Sequenz zu analysierendes DNA-Molekül über einen solchen nicht-kovalenten DNA-Linker zum einen an einen festen Träger immobilisiert, und zum anderen an ein signalverstärkendes branchedDNA-Molekül (bDNA) gebunden werden soll (siehe Fig. 3 in S. Urdea, Bio/Technol. 1994, 12, 926 oder US-Patent Nr. 5,624,802). Ein wesentlicher Nachteil der zuletzt beschriebenen Systeme ist, daß sie den Verfahren zur Nucleinsäure-Diagnostik durch Polymerase-Chain-Reaction (PCR) (K. Mullis, Methods Enzymol. 1987, 155, 335) hinsichtlich der Empfindlichkeit bis jetzt unterlegen sind. Das ist u. a. darauf zurückzuführen, daß die nicht-kovalente Bindung vom festen Träger an das zu analysierende DNA-Molekül ebenso wie die nicht-kovalente Bindung des zu analysierenden DNA-Moleküls nicht immer spezifisch erfolgt, wodurch es zu einer Vermischung der Funktionen "Sequenzerkennung" und "nicht-kovalente Bindung" kommt. Die Verwendung von p-NA's als orthogonales Paarungssystem, welches nicht in das DNA- bzw. RNA-Paarungsgeschehen eingreift, löst dieses Problem auf vorteilhafte Weise, wodurch die Empfindlichkeit der beschriebenen analytischen Verfahren deutlich erhöht werden kann.

Ein weiterer Gegenstand der vorliegenden Erfindung ist daher ein Konjugat enthaltend ein erfindungsmäßiges 3'-Desoxypentopyranosyl-Nucleosid der Formel (I) oder (II) und ein Biomolekül, ausgewählt aus der Gruppe der Peptide, Proteine und Nucleinsäuren.

Konjugate sind im Sinne der vorliegenden Erfindung kovalent gebundene Hybride aus p-NA's und einem Peptid, Protein oder einer Nucleinsäure, beispielsweise ein Antikörper oder ein funktioneller Teil davon oder eine in ihrer natürlichen Form vorkommende DNA und/oder RNA. Funktionelle Teile von Antikörper sind beispielsweise Fv-Fragmente (Skerra & Plückthun (1988) Science 240, 1038), einzelkettige Fv-Fragmente (scFv; Bird et al. (1988), Science 242, 423; Huston et al. (1988) Proc. Natl. Acad. Sci. U.S.A., 85, 5879) oder Fab-Fragmente (Better et al. (1988) Science 240, 1041).

In einer bevorzugten Ausführungsform handelt es sich dabei um p-DNA/DNA- bzw p-DNA/RNA-Konjugate.

Konjugate werden dann vorzugsweise verwendet, wenn die Funktionen "Sequenzerkennung" und "nicht-kovalente Bindung" in einem Molekül realisiert werden müssen, da die erfindungsgemäßen Konjugate zwei zueinander orthogonale Paarungssysteme enthalten.

Für die Herstellung von Konjugaten sind sowohl sequentielle als auch konvergente Verfahren geeignet.

In einem sequentiellen Verfahren wird z. B. nach erfolgter automatisierter Synthese eines p-RNA-Oligomeren direkt am gleichen Synthesizer - nach Umstellung der Reagenzien und des Kupplungsprotokolls - z. B. ein DNA-Oligonukleotid weitersynthetisiert. Dieser Vorgang läßt sich auch in umgekehrter Reihenfolge durchführen.

In einem konvergenten Verfahren werden z. B. p-RNA-Oligomere mit Aminoterminalen-Linkern und z. B. DNA-Oligomere mit z. B. Thiol-Linkern in getrennten Vorgängen synthetisiert. Anschließend erfolgt vorzugsweise eine Jodacetylierung des p-DNA-Oligomeren und die Kupplung der beiden Einheiten nach literaturbekannten Protokollen (T. Zhu et al., Bioconjug. Chem. 1994, 5, 312).

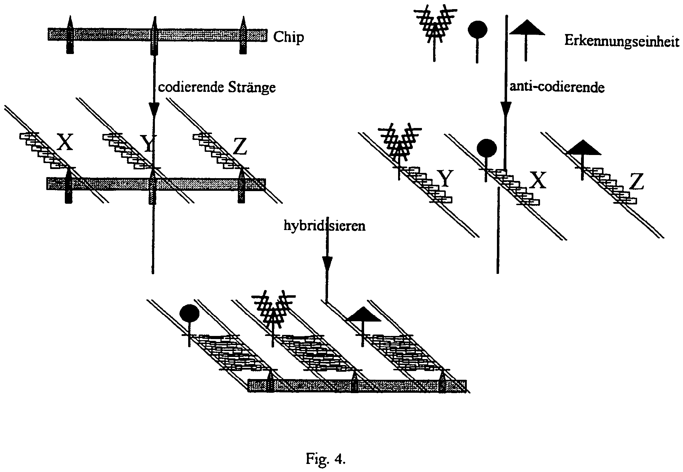

Konvergente Verfahren erweisen sich aufgrund ihrer Flexibilität als besonders bevorzugt. Unter dem Begriff Konjugat im Sinne der vorliegenden Erfindung sind auch sogenannte Arrays zu verstehen. Arrays sind Anordnungen von immobilisierten Erkennungsspecies, die speziell in der Analytik und Diagnostik eine wichtige Rolle bei der simultanen Bestimmung von Analyten spielen. Beispiele sind Peptide-Arrays (Fodor et al., Nature 1993, 364, 555) und Nucleinsäure-Arrays (Southern et al. Genomics 1992, 13, 1008; Heller, US-Patent Nr. 5,632,957). Eine höhere Flexibilität dieser Arrays kann dadurch erreicht werden, daß die Erkennungsspecies an codierende Oligonucleotide gebunden werden und die zugehörigen, komplementären Stränge an bestimmte Positionen auf einem festen Träger. Durch Aufbringen der codierten Erkennungsspecies auf den "anti-codierten" festen Träger und Einstellung von Hybridisierungsbedingungen werden die Erkennungsspecies an den gewünschten Positionen nicht-kovalent gebunden. Dadurch können verschiedene Typen von Erkennungsspecies, wie z. B. DNA-Abschnitte, Antikörper, nur durch Anwendung von Hybridisierungsbedingungen gleichzeitig auf einem festen Träger angeordnet werden (siehe Fig. 4.). Voraussetzung hierzu sind aber äußerst starke, selektive - um die codierenden Abschnitte möglichst kurz zu halten - und mit natürlicher Nucleinsäure nicht interferierender Codons und Anticodons notwendig. p-NA's, vorzugsweise p-DNA's eignen sich hierzu in besonders vorteilhafter Weise.

Die vorliegende Erfindung ermöglicht daher auch ein Verfahren, mit denen Erkennungsspecies, bevorzugt natürliche DNA- oder RNA-Stränge und Proteine, dabei bevorzugt Antikörper oder funktionelle Teile von Antikörper, durch p-NA-Abschnitte, bevorzugt p-DNA-Abschnitte, eindeutig codiert werden. Diese können dann gemäß Fig. 4. mit den zugehörigen Codons auf einem festen Träger hybridisiert werden. Damit kann auf einem festen Träger, der in Form eines Arrays mit Codons ausgestattet ist, nur durch Einstellung von Hybridisierungsbedingungen mit immer neuen Kombinationen von Erkennungsspecies an den gewünschten Positionen immer neue, diagnostisch nützliche Arrays aufgebaut werden. Wird dann der Analyt, beispielsweise eine biologische Probe wie Serum o. ä. aufgebracht, dann werden die zu detektierenden Species in einem bestimmten Muster auf dem Array gebunden, welches dann indirekt (z. B. durch Fluoreszenzmarkierung der Erkennungsspecies) oder direkt (z. B. durch Impedanzmessung am Anknüpfungspunkt der Codons) registriert wird. Dann wird die Hybridisierung durch geeignete Bedingung aufgehoben (Temperatur, Salze, Lösungsmittel, elektrophoretische Vorgänge) so daß wieder nur der Träger mit den Codons zurückbleibt. Dieser wird dann erneut mit anderen Erkennungsspecies beladen und wird z. B. für den gleichen Analyten für die Ermittlung eines anderen Musters verwendet. Die immer neue Anordnung von Erkennungsspecies im Array-Format und die Verwendung von p-NA's als Paarungssysteme ist gegenüber anderen Systemen, siehe z. B. WO 96/13522 (s. 16, unten), besonders vorteilhaft.

Die folgenden Figuren und Beispiele sollen die Erfindung näher beschreiben, ohne sie zu beschränken.

### BESCHREIBUNG DER FIGUREN

Fig. 1 zeigt die Synthese der Zuckerkomponente, wobei T Triphenylmethyl (Trityl) bedeutet.

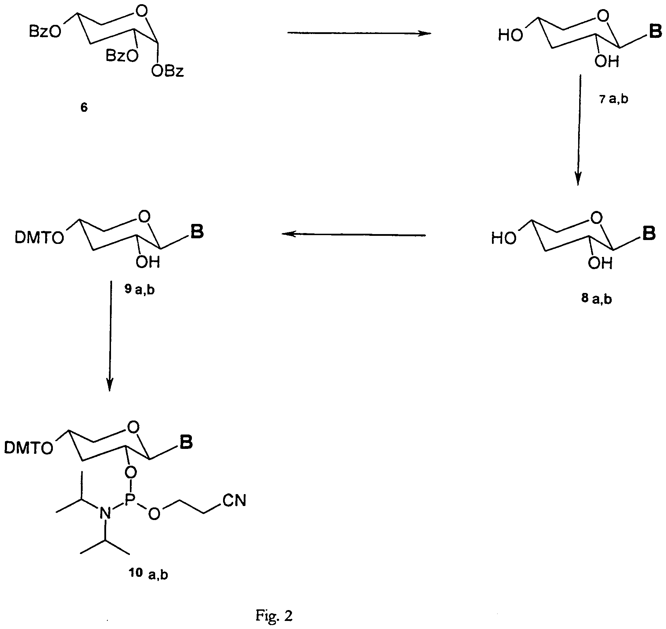

Fig. 2 zeigt den Syntheseweg zu den monomeren Bausteinen, wobei B eine in der Natur vorkommende oder synthetische Nucleobase bedeutet.
Fig. 3 zeigt einen Ausschnitt aus der Struktur von RNA in ihrer natürlich vorkommenden Form (links) und eine p-DNA (rechts).
Fig. 4 zeigt schematisch eine Anordnung von immobilisierten Erkennungsstrukturen (Arrays) auf einem festen Träger.

### BEISPIELE

### Beispiel 1

### 1,2-O-Isopropyliden-3-O-[(methylthio)thiocarbonyl]-5-O-trityl-α-D-xylofuranose (3)

444,3 g (1,027 mol) 1,2-O-Isopropyliden-5-O-trityl-α-D-xylofuranose (2)wurden in 1000 ml abs DMF gelöst. Unter Kühlung, KPG-Rührung, N₂ -Atmosphäre und leichtem N₂-Strom bei 0-5° C wurden portionsweise innerhalb von 1 h 29,58 g (1,232 mol) NaH zugegeben. Nach beendeter NaH Zugabe wurde noch weitere 15 Minuten unter Kühlung gerührt. Man rührte noch eine weitere Stunde ohne Kühlung bis kein Wasserstoff mehr entsteht. Die Innentemperatur der klaren Lösung betrug 12° C. Nun wurden 57,7 ml (1,027 mol) Schwefelkohlenstoff innerhalb von 20 Minuten zugetropft. Die Reaktionstemperatur wurde durch Kühlung bei 20-25° C gehalten. Nach 30 Minuten wurden 77,4 ml (1,027 mol) Jodmethan innerhalb von 20 Minuten unter leichter Kühlung langsam zugegeben (T=20-25°C). Während der Reaktion mußten weitere 500 ml DMF zugegeben werden. Nach weiteren 2 h wurde der Ansatz auf 2 1 Eiswasser und 1,5 1 Dichlormethan gegeben und ausgerührt. Die org. Phase wurde mit 4 x 0,7 1 Wasser extrahiert, über Na₂SO₄ getrocknet, filtriert und eingeengt. Man erhielt 610.5 g rohes Produkt welches direkt weiter umgesetzt wurde.
DC (Kieselgel, Aceton/Heptan 1:4): R_{f}=0.30.
¹H NMR (300 MHz, CDCl₃): 1,32, 1,56 (2s, 3H, 2 x CH₃), 2,41 (s, 3H, S-CH₃), 3,32 (dd, 1H, H-C(5)), 3,47 (dd, 1H, H-C(5)), 4,54 (m, 1H, H-C(4)), 4,64 (m, 1H, H-C(2)), 5,89 (m, 1H, H-C(3)), 6,09 (d, J = 3 Hz, 1H, H-C(1)), 7,18-7,43 (m, 15H, Hₐᵣₒₘ).

### Beispiel 2

### 3-Desoxy-1,2, 4-Tri-O-Benzoyl-α-D-erythro-pentose (6)

4,16 g (1 mmol) 3-Desoxy-1,2-O-Isopropyliden-5-O-trityl-_{α}-D-xylofuranose (4) wurden in 20,0 ml Dichlormethan gelöst. Unter Rühren bei RT wurden 20,0 ml Wasser und 2,0 ml Trifluoressigsäure zugegeben und 17 h bei RT gerührt. Die Phasen wurden getrennt, die wässrige Phase wurde 2 x mit je 20 ml Dichlormethan extrahiert und etwas eingeengt. Der Rückstand wurde noch 2x in jeweils 20 ml Wasser gelöst und eingeengt. Der Rüchstand wurde wieder in 20 ml Wasser gelöst, mit 2,0 g stark basischem Ionentauscher 15 Minuten verrührt (pH-Wert 7-8), der Ionentauscher abfiltriert und zur Trockne eingeengt. Das leicht gelbliche Öl wurde mit 27 ml abs. Pyridin/Dichlormethan 2:1 versetzt, 2.0 g Molsieb 4 Å addiert und 1 h unter Argon gerührt. Nun wurden 6.1 ml Benzoylchlorid in 6.1 ml abs. Pyridin bei -30°C zugetropft. Nach 1 Stunde ließ man auf Raumtemperatur kommen und rührte über Nacht. Nach der Zugabe von 2 ml MeOH wurde eingeengt, der feste Rückstand mit Toluol koevaporiert, abermals in Toluol aufgenommen, verrührt und abfiltriert. Das Filtrat wurde eingeengt und über eine Kieselgelsäule (Kieselgel 60, 4 x 33 cm) mit einem linearen Gradienten von Heptan zu Heptan/EtOAC 2:1 in 41 gereinigt. Die erhaltenen Produktfraktionen wurden einegeengt, in 30 ml Diethylether verrührt und der Feststoff abgesaugt. Man erhielt 1.07 g (25%) eines weißen Feststoffs.
DC (Kieselgel, Dichlormethan/MeOH 4:1): R_{f}=0.45.
DC (Kieselgel, Heptan/EtOAc 2:1): R_{f}=0.35.
¹H NMR (300 MHz, CDCl₃): 2,51 (m,2H,H-C(3)), 4,01 (dd,1H,H-C(5)), 4,21 (dd,1H,H-C(5)), 5,17 (m,2H, H-C(4), H-C(2)), 6,38 (d, J=2 Hz,1H,H-C(1)), 7,18-7.95 (m,16 H,Hₐᵣₒₘ), 7,40 (m.4H,Hₐᵣₒₘ), 7,52 (m,1H,Hₐᵣₒₘ), 7,95 (m,6H,Hₐᵣₒₘ).
¹³C (300 MHz, CDCl₃):
27,28 (s, C(3)), 63,45 (s, C(5)), 65,56 (s, C(4)), 66,24 (s, C(2)), 91,202 (s, C(1)), 128,2-129,9 (m, 12Cₐᵣₒₘ), 133,07 (s, C_{arom, para}), 133,22(s, C_{arom, para}), 133,71 (s, C_{arom, para}), 164,22 (s, C=O), 165,60 (s, C=O), 166,06 (s, C=O).

### Beispiel 3

### Synthese des 1-{3'-Desoxy-4'-O-[(4,4'-dimethoxytriphenyl)-methyl]-β-D-ribopyranosyl}-thymin-2'-O-(2-cyanoethyl-N,N-disopropyl)-phosphoramidits (10a)

### Synthese des 1-(3'-Desoxy-2,4-Di-O-benzoyl-_{β}-D-ribopyranosyl)-thymins (7a)

1,0 g (2,24 mmol) 3'-Desoxy-1,2,4-Tri-O-benzoyl-ribopyranose (6) und 283 mg (2,24 mmol) Thymin wurden in 11,0 ml Acetonitril suspendiert und auf 60°C erwärmt. Zu dieser Mischung wurden innerhalb von 10 Minuten 957 mg (4,7 mmol) N,O-Bis-(trimethylsilyl) acetamid (BSA) mit einer Spritze zugetropft und 15 min bei 60°C belassen. Zu der entstandenen Lösung addiert man 2,02 g (9,07 mmol) Trifluormethansulfonsäuretrimethylsilylester (=TMS-triflat) innerhalb von 45 min und rührt 2 h bei 60°C nach. Das Reaktionsgemisch läßt man auf RT abkühlen, verdünnt mit EtOAc und extrahiert gegen verdünnte NaHCO₃-Lösung. Die EtOAc-Phase wurde nochmals mit Wasser extrahiert, über Natriumsulfat getrocknet, filtriert und eingeengt. Durch Chromatographie an Kieselgel 60 (3 x 28 cm) mit einem linearen Gradienten von Heptan bis Heptan/EE = 1/1 in 41, erhielt man nach Einengen der produkthaltigen Fraktionen einen farblos amorphen Feststoff, der in 20 ml Diethylether aufgenommen und verrührt wurde. Es resultierten 1.0 g (99%) des gewünschten Produkts.
DC (Kieselgel, EtOAc/Heptan 1:1): R_{f}=0.27. ¹H NMR (300 MHz, CDCl₃):
1,94 (d, 3H, CH₃), 2.12 (dd, 1H, H_{eq}-C(3')), 2,93(m, 1H, Hₐₓ-C(3')), 3,70(t, 1H, H_{eq}-C(5')), 4,40 (m, 1H, Hₐₓ-C(5')), 5,25 (m, 1H, H-C(4')), 5,32 (m, 1H, H-C(2')), 5,93 (d, J=9,3 Hz, 1H, H-C(1')), 7,22(d, 1H, H-C(6)) 7,38-7,50 (m, 4H, 3,5-Hₐᵣₒₘ), 7,54-763 (m, 2H, 4-Hₐᵣₒₘ), 7,94-8,04 (m, 4H, 2,6-Hₐᵣₒₘ), 8,26(bs, 1H, H-N(3)).
¹³C (300 MHz, CDCl₃): 12,52 (CH₃), 165,10 (C=O), 34,63 (C(3')), 65,85 (C(4')), 67,64 (C(2')), 69,16 (C(5')), 82,22 (C(1')), 111,94 (C(5)), 128,50 (Cₐᵣₒₘ), 128,54 (Cₐᵣₒₘ), 128,72 (Cₐᵣₒₘ), 129,21 (Cₐᵣₒₘ), 129,71 (Cₐᵣₒₘ), 129,85 (Cₐᵣₒₘ), 133,51 1(Cₐᵣₒₘ), 133,63 (Cₐᵣₒₘ), 134,66 (C(6)), 150,69 (C(2)), 163,31 (C(4)), 165,34 (C=O).

### Synthese des 1-(3'-Desoxy-ribopyranosyl)-thymins (8a)

807 mg (1,79 mmol) 1 wurden in 20 ml methanolischem Ammoniak 48 h bei RT gerührt. Danach wurde die Reaktionslösung eingeengt, der feste Rückstand in EtOAc/MeOH = 9:1 verrührt und abgesaugt. Man erhielt 345 mg (80%) eines weißen kristallinen Feststoffs. Die Mutterlauge wurde eingeengt und über eine Kieselgelsäule (Kieselgel 60, 3 x 15 cm)mit einem linearen Gradienten von EtOAc bis EtOAc/MeOH = 9:1 in 31 gereinigt. Man erhielt weitere 70 mg (16%) des gewünschten Produkts. Insgesamt wurden 415 mg (96%) von ₂ isoliert.
DC: (Kieselgel, EtOAc/MeOH 9:1) R_{f} = 0,28
¹H-NMR (300 MHz, MeOD): 1,48 (q, 1H, H_{b}( 3'), 1,80 (d, 3H, CH₃), 2,37 (m, 1H, H_{b}(3')), 3,20 (t, 1H, H_{b}(5')), 3,24 (m, 2H, OH), 3,70 (m, 1H, H(4')), 3,75 (m, 1H, H(2')), 3,90 (ddd, 1H, Hₐ(5')), 5,22 (d, 1H, J=9 Hz, H(1')), 7,37 (d, 1H, H(6)).

### Synthese des 1-{3'-Desoxy-4'-O-[(4,4'-dimethoxytriphenyl)-methyl]-β-D-ribopyranosyl}-thymins (9a)

320 mg (1,32 mmol) 8a wurden unter N₂-Atmosphäre in 6 ml abs. Dichlormethan/Pyridin 1:2 gelöst, 1 g Molsieb 4 Å addiert und der Ansatz 15 min bei RT gerührt. Nun kühlte man auf - 10°C ab, addierte 0,47 ml Diisopropylamin und 0,76 g ( 2,24 mmol) Dimethoxytrityl-chlorid (DMTCI) ließ auf RT kommen. Es wurde über Nacht gerührt. Nochmalige Zugabe von 0,38 g (1,12 mmol) DMTCl in 2 ml abs. Dichlormethan und rühren über Nacht brachte die Reaktion zur Vollständigkeit. Der Ansatz wurde vom Molsieb abgesaugt, auf halbgesättigte NaHCO₃-Lösung gegossen und mit Methylenchlorid extrahiert. Die Methylenchloridphase wurde 2x mit Wasser ausgeschüttelt, über Na₂SO₄ getrocknet, filtriert und eingeengt. Es wurde über eine Kieselgelsäule (Kieselgel 60; 3 x 20 cm) und einem Gradienten (2 1 n-Heptan und 21 n-Heptan/ EE = 1/1als linearer Gradient) gereinigt. Zum Schluß wurde die Säule mit Heptan/EtOAc/MeOH 5:5:1 gewaschen, die produkthaltigen Fraktionen einrotiert, in 50 ml Tetrachlorkohlenstoff verrührt und wiederum eingeengt. Der Rückstand wurde an der HV über Nacht getrocknet. Man erhielt 352 mg (32%) des zweifach tritylierten Produkts 12a. Die polaren Fraktionen wurden noch einmal über eine Kieselgelsäule (Kieselgel 60, 3 x 25 cm) mit einem linearen Gradienten von Dichlormetan bis Dichlormethan/MeOH 19=1 in 41 aufgetrennt. Man isolierte 50 mg (7%) an 1-{3'-Desoxy-2'-O-[(4,4'-dimethoxytriphenyl)-methyl]-_{β}-D-ribopyranosyl}-thymin 11a und 293 mg (41%) 9a. DC (Kieselgel):
9a(CH₂Cl₂/MeOH 19:1) Rf=0,26
11a (CH₂Cl₂/MeOH 19:1) Rf=0,29
12a (EtOAc/Heptan 4:1) R_{f}=0,49
¹H-NMR (300 MHz, CDCl₃): 1,75 (s, 3H, CH₃), *2,20 (m, 1H, Hₐₓ(3')),* 2,92 *(m, 1H, H_{eq}(3'))*, *3,11 (m, 2H, H(4'), Hₐₓ-C(5')),* 3,34 *(m, 1H, H_{eq}(5')),* 3,64 *(m, 1H H(2')),* 3,71 (d, 6H, 2 x OCH₃), 5,26 (d, 1H, J=9 Hz, H(1')), 6,76 (m, 4H, Hₐᵣₒₘ), 6,89 (d, 1H, H(6)), 7,10-7,44 (m, 9H, Hₐᵣₒₘ), 9,14(bs, 1H, H-N(3))

### Synthese des 1-{3'-Desoxy-4'-O-[(4,4'-dimethoxytriphenyl)-methyl]-β-D-ribopyranosyl}-thymin-2'-O-(2-cyanoethyl-N.N-disopropyl)-phosphoramidits (10a)

218 mg (0,4 mmol) 1-{3'-Desoxy-4'-O-[(4,4'-dimethoxytriphenyl)-methyl]-β-D-ribopyranosyl}-thymin 9a wurden in 2,0 ml abs Dichlormethan gelöst und mit 155 mg (1,2 mmol) N-Ethyldiisopropylamin versetzt. Bei RT. wurden nun 237 mg (1,0 mmol) Phosphorigsäure-mono-(2-cyanoethylester)diisopropyl-amidchlorid innerhalb von zwei Minuten zugetropft. Der Ansatz rührte 3 h bei RT., wurde mit CH₂Cl₂ auf 40 ml verdünnt und mit 50 ml Phosphatpuffer (pH=7) extrahiert, die org. Phase über Na₂SO₄ getrocknet, filtriert und eingeengt. Das Rohprodukt wurde über eine Kieselgelsäule (3 x 15 cm) mit einem linearen Gradienten von EE/Heptan = 1:2 bis EE in 41 gereinigt. Man erhielt 266 mg (89%) eines farblosen Harzes.
DC (Kieselgel, Heptan/EtOAc 4:1): R_{f} = 0,47/0.54
¹H-NMR (400 MHz in CDCl₃):
1,10 (m, 6H, 2x CH₃); 1,83 (m, 1H, Hₐₓ-C(3')); 1,88 (m, 3H, CH₃-C(5)); 2,21 (m, 1H, H_{eq}-C(3')); 2,41-2,61 (m, 2H, CH₂CN); 2,98 (m, 1H, H_{eq}-C(5')); 3,19 (m, 1H, Hₐₓ-C(5')); 3,35-3,80 (m, 6H, CH₂OP, H-C(4'), H-C(2'); 2xCH); 3,8 (m, 6H, 2xCH₃); 5,41 (d, J=8,86 Hz, 1H, H-C(1')); 6,8 (m, 4H, H_{DMT}); 6,97 (m, 1H, H-C(6)); 7,20-7,52 (m, 9H, Hₐᵣₒₘ); 8,25 (s,1H, H-N(3)).

### Beispiel 4

### Synthese des N⁶-Benzoyl-3-{3-Desoxcy-4'-O-[(4,4'-dimethoxytriphenyl)-methyl]-β-D-ribopyrano-syl}-adenins-2'-O-(2-cyanoethyl-N,N-disopropyl)-phosphoramidits (10b)

### Synthese des N⁶-Benzoyl-3-(3'-desoxy-2,4-di-O-benzoyl-ribopyranosyl)-adenins (7b)

2,23 g (5 mmol) 6 und 1,20 g (5 mmol) N⁶-Benzoyladenin wurden unter Argonatmosphäre in 35 ml abs. Acetonitril vesetzt und 15 Minuten gerührt. Es wurde auf leichten Rückfluß erhitzt. Zu dieser Suspension wurde innerhalb von 20 Minuten 2,14 g (10,5 mmol) BSA zugetropft und 15 min bei 68°C gerührt. Nun wurden innerhalb 5 Minuten 4,7 g (18 mmol) Zinntetrachlorid zugetropft und 1h am Rückfluß gerührt. Man ließ auf RT kommen, goß auf 150 ml gesättigter NaHCO₃-Lsg und extrahierte gegen 100 ml EtOAc. Der ausgefallene Niederschlag wurde abgesaugt und mit 150 ml EtOAc gewaschen. Die org. Phase wurde nochmals mit 100 ml Wasser ausgeschüttelt, über Natriumsulfat getrocknet, filtriert und eingeengt. Man reinigte über eine Kieselgelsäule (3 x 26 cm) mit einem Gradienten (21 EtOAc/Heptan 2:1 und 21 EtOAc linearer Gradient), vereinigte die produkthaltigen Chargen und isolierte 2,7 g (96 %) eines weißen Feststoffs.
DC (Kieselgel): 4 (EtOAc): R_{f}= 0,40.
¹H-NMR (300 MHz, CDCl₃): 2,16 (m, 1H, Hₐₓ(3')), 2,97 (m, 1H, H_{eq}(3')), 3,76 (m, 1H, Hₐₓ(5')), 4,41 (m, 1H, H_{eq}(5')), 5,38 (m, 1H, H(4')), 5,62 (m, 1H, H(2')), 5,98 (d, 1H, J = 8,92 Hz, H(1')), 7,22-8,0 (m, 15H, Hₐᵣₒₘ), 8,21 (s, 1H, H(8)), 8,74 (s, 1H, H(2)), 8,91 (bs, 1H, NH).
¹³C-NMR (300 MHz, CDCl₃): 34,57 (C(3')), 65,80 (C(4')), 68,86 (C(2')), 69,13 (C(5')), 82,67 (C(1')), 122,53 (C(6)), 127,76-133,62 (12 x C(arom)), 140,62 (C(8)), 149,63 (C(6)), 151,91 (C(4)), 153,03 (C(2)), 164,44 (C=O an N-C(6)), 164,77 (C=O), 165,32 (C=O).

### Synthese des N⁶-Benzoyl-3-(3'-Desoxy-β-D-ribopyranosyl)-adenins (8b)

280 mg (0,5 mmol) 7b wurden in 7 ml THF/MeOH/H₂O 5:4:1 gelöst und, auf -5°C abgekühlt und 2,22 ml 32 %ige NaOH-Lösung in THF/MeOH/H2O 5:4:1 langsam zugegeben, so daß die Temp. unter 0°C blieb. Man rührte 20 min unter Kühlung, versetzte mit 400 mg (7,5 mmol) Ammoniumchlorid und ließ die Lösung auf RT kommen. Die Lösungsmittel wurden abgezogen, der Rückstand in 20ml MeOH gelöst, auf 10 g Kieselgel aufgezogen und chromatographisch über eine Kieselgelsäule (3 x 12 cm mit 1 1 Dichlormethan und 2 1 CH₂Cl₂/MeOH = 4/1 als linearer Gradient) gereinigt. Es wurden 157 mg (88%) des farblosen Feststoffs 8b isoliert.
DC (Kieselgel): 5 (EtOAc/MeOH 4:1) R_{f} = 0,34 ¹H-NMR (300 MHz, MeOD): 1,58 (q, 1H, Hₐₓ(3')), 2,45 (m, 1H, H_{eq}(3')), 3,33 (m, 1H, Hₐₓ(5')), 3,87 (m, 1H, H(4')), 3,97 (m, 1H, H_{eq}(5')), 4,25 (m, 1H, H(2')), 5,40 (d, 1H, J=9,2 Hz, H(1')), 7,30-8,02 (m, 5H, Hₐᵣₒₘ), 8,47 (s, 1H, H(8)), 8,63 (s, 1H, H(2)).
Synthese des N⁶-Benzoyl-3-{3'-desoxy-4'-O-[(4,4'-dimethoxytriphenyl)-methyl]-β-D-ribopyranosyl}-adenins (9b)

In einer Argonatmosphäre wurden 1,02 g (2,87 mmol) 8b in 9,0 ml abs Pyridin gelöst, 1,56 g (12 mmol) N-Ethyldiisopropylamin und 1,0 g Molsieb 4Å addiert und 30 min bei RT gerührt. Nun wurde auf -10°C abgekühlt, 2,2 g (6,49 mmol) DMTCl in 5,0 ml abs. Chloroform gelöst innerhalb von 30 min zugetropft. Der Versuch rührte bei RT über Nacht. Nach 22 h wurden wiederum 200 mg (0,59 mmol) DMTCl und 2 h darauf weitere 430 mg (1,27 mmol) DMTCl in fester Form zugegeben. Nach nochmals 22 h bei RT wurde der Ansatz auf 100 ml einer halbgesättigten NaHCO₃-Lsg. gegossen, mit 100 ml Methylenchlorid versetzt und extrahiert. Die org. Phase wurde noch 2 x mit je 100 ml H₂O rückextrahiert, über Na₂SO₄ getrocknet, filtriert und eingeengt. Reinigung über eine Kieselgelsäule(3 x 25 cm) mit einem Gradienten (21 EtOAc/Heptan 2:1 und 21 EtOAc mit einem linearen Gradienten) lieferte: 520 mg (27,5 %) 9b, 430 mg (23%) Mischung aus 9b und 116, 370 mg (13,4 %) 12b und 370 mg (20%) des Edukts.
DC (Kieselgel, EtOAc):
9b: R_{f} = 0.29
11b: R_{f}=0.12
12b: R_{f}= 0.55 ¹H-NMR ( 500 MHz in CDCl₃):
1,92 (m, 1H, Hₐₓ(3')), 2,42 (m, 1H, H_{eq}(3')), 2,99 (m, 1H, H_{eq}(5')), 3,21 (m, 1H, Hₐₓ(5')), 3,79 (d, 6H, 2 x OCH₃), 3,84 (m, 1H, H(4')), 4,13 (m, 1H, H(2')), 5,17 (bs, 1H, OH), 5,32 (d, 1H, J=8,7 Hz, H(1')), 6,86 (dd, 4H, Hₐᵣₒₘ), 7,20-7,75 (m, 12H, Hₐᵣₒₘ), 7,96 (m, 3H, 1H(8), 2Hₐᵣₒₘ), 8,54 (s, 1H, H(2)), 8,94 (bs, 1H, NH).
¹³C-NMR (500 MHz in CDCl₃)
39,13 (C(3')), 55,26 (2 x OCH₃), 66,59 (C(4')), 67,72 (C(2')), 70,76 (C(5')), 86,69 (C_{tert Trityl}), 86,93 (C(1')), 113,31 (2Cₐᵣₒₘ), 113,34 (2Cₐᵣₒₘ₎, 121,8 (C(5)), 127,04 - 136,74 (11Cₐᵣₒₘ), 141,74 (C(8)), 145,51 (Cₐᵣₒₘ), 148,85 (C(6)), 151,56 (C(2)), 158,74 (2Cₐᵣₒₘ), 164,61 (C=O).

### Synthese des N⁶-Benzoyl-3-{3'-Desoxy-4'-O-[(4,4'-dimethoxytriphenyl)-methyl]-β-D-ribopyrano-syl}-adenins-2'-O-(2-cyanoethyl-N,N-disopropyl)-phosphoramidits (10b)

380 mg (0,58 mmol) N⁶-Benzoyl-3-{3'-desoxy-4'-O-[(4,4'-dimethoxytriphenyl)-methyl]-β-D-ribopyra-nosyl}-adenin 9b wurden in 2,0 ml abs Dichlormethan gelöst und mit 224 mg (1,73 mmol) N-Ethyldiisopropylamin versetzt. Bei RT wurden nun 342 mg (1,44 mmol) Phosphorigsäure-mono-(2-cyanethylester)diisopropylamid-chlorid innerhalb von zwei Minuten zugetropft. Der Ansatz rührte 3 h bei RT, wurde mit CH₂Cl₂ auf 40 ml verdünnt und mit 50 ml Phosphatpuffer (pH=7) extrahiert. Die org. Phase wurde über Na₂SO₄ getrocknet, filtriert und eingeengt. Man reinigte Kieselgelsäule (3 x 15 cm) mit einem linearen Gradienten von EtOAC/Heptan (1:2) bis EtOAc/Heptan (4: 1) als lineraren Gradienten. Es wurden 400 mg (80%)eines gelblichen Schaumes erhalten.
DC (Kieselgel, EtOAc/Heptan 4:1): Rₜ = 0,38
¹H-NMR (400 MHz in CDCl₃):
1,05 (m, 6H, 2xCH₃); 1,87 (m, 1H, Hₐₓ-C(3')); 2,23 (m, 1H, H_{eq} -C(3')); 2,32 & 2,55 (2 x m, 2H, CH₂CN); 3,05-3,70 (m, 6H, 2xCH, CH₂OP, 2xH-C(5')); 3,79 (m, 6H, 2xOCH₃); 3,90 (m, 1H, H-C(4')); 4,12 (m, 1H, H-C(2')); 5,47 (2xd, J=8,87Hz, 1H, H-C(1')); 6,85 (m, 4H, H_{DMT}); 7,20-7,65 (m, 13 H, Hₐᵣₒₘ); 8,0 (m, 2H, Hₐᵣₒₘ); 8,09 (s, 1H, H-C(8)); 8,80 (s, 1H, H-C(2)); 8,97 (s, br, 1H, HN)

### Synthese des N⁶-Benzoyl-3-{3'-desoxy-4'-O-[(4,4'-dimethoxytriphenyl)-methyl]-β-D-ribopyranosyl}-adenin-2'-O-succinoyls (13b)

115 mg (0,174 mmol; 1 eq) von N⁶-Benzoyl-3-{3'-desoxy-4'-O-[(4,4'-dimethoxytriphenyl)-methyl]- β-D-ribopyranosyl}-adenins (9b) wurden zusammen mit 35mg (0,35 mmol; 2 eq) Bernsteinsäure-anhydrid und 25 mg (0,21 mmol; 1,2 eq) DMAP in 1,0 ml abs CH₂Cl₂ unter N₂-Atmosphäre 2 ½ h bei RT gerührt. Dann wurde mit Methylenchlorid auf 20 ml verdünnt, 1x mit 20 ml 10%iger Zitronensäure und 3x mit je 20 ml Wasser extrahiert. Die org. Phase wurde über Natriumsulfat getrocknet, filtriert und eingeengt. Man erhielt 130 mg (99 %) von 13b.
DC (Kieselgel, EtOAc/MeOH 19:1): R_{f}= 0,14 ¹H-NMR (500 MHz, CDCl₃ ):
1,82 (m, 1H, Hₐₓ(3')), 2,20 (m, 2H, 2 x CH₂), 2,32 (m, 1H, H_{eq}(3')), 3,02 (m, 1H, H_{eq}(5')), 3,30 (m, 1H, Hₐₓ(5')), 3,73 (d, 6H, 2 x OMe), 3,82 (m, 1H, H(4)), 5,06 (m, 1H, H(2')), 5,55 (d, J = 9,5 Hz, 1H, H(1')), 6,79 (m, 4H, o zu OMe), 7,14-7,49 (m, 12H, 9H_{Trityl}, 3H_{Bz}), 7,87-7.91 (m, 2H, 2H_{Bz}), 7,94 (s, 1H, H-C(8)), 8,55 (s, 1H, H(2)), 9,6 (bs, 1H, -COOH).

## Patentansprüche

1. 3'-Desoxypentopyranosyl-Nucleosid-Bausteine der Formel (I), 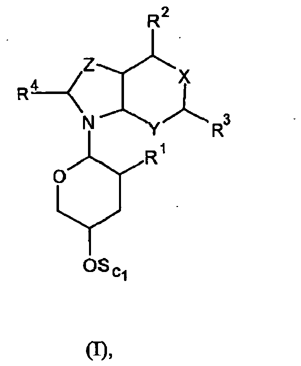 worin
R¹ gleich OH, Hal mit Hal gleich Br oder Cl, ein Rest ausgewählt aus 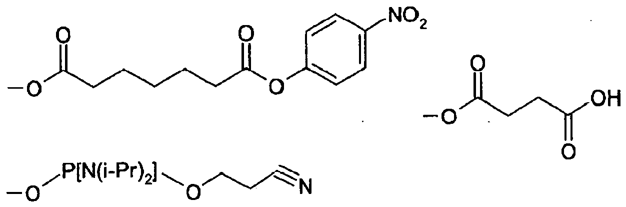 mit i-Pr gleich Isopropyl, oder -O-PH-(=O)(-O⁻), R², R³ und R⁴ unabhängig voneinander, gleich oder verschieden, jeweils H, NR⁵R⁶, OR⁷, SR⁸, =O, CₙH₂ₙ₊₁ mit n eine ganze Zahl von 1-12, vorzugsweise 1-8, insbesondere 1-4, eine ß-eliminierbare Gruppe der Formel -OCH₂CH₂R¹⁸ mit R¹⁸ gleich ein Cyano- oder p-Nitrophenylrest oder ein Fluorenylmethyloxycarbonyl-(Fmoc)-Rest, oder (CₙH₂ₙ)NR¹⁰R¹¹, mit R¹⁰R¹¹ gleich H, CₙH₂ₙ₊₁ oder R¹⁰R¹¹ verbunden über einen Rest der Formel 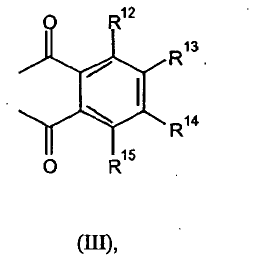 worin R¹², R¹³, R¹⁴ und R¹⁵ unabhängig voneinander, gleich oder verschieden, jeweils H, OR⁷, oder CₙH₂ₙ₊₁, oder CₙH₂ₙ₋₁,wobei n die oben genannte Bedeutung hat, bedeuten und
R⁵, R⁶, R⁷ und R⁸ unabhängig voneinander, gleich oder verschieden, jeweils H, CₙH₂ₙ₊₁, oder CₙH₂ₙ₋₁, wobei n die oben genannte Bedeutung hat, -C(O)R⁹ mit R⁹ gleich ein linearer oder verzweigter, gegebenenfalls substituierter Alkyl-, Aryl-, vorzugsweise Phenyl-Rest,
X, Y und Z unabhängig voneinander, gleich oder verschieden, jeweils =N-, =C(R¹⁶)- oder -N(R¹⁷)- mit R¹⁶ und R¹⁷ unabhängig voneinander, gleich oder verschieden, jeweils H oder CₙH₂ₙ₊₁ oder (CₙH₂ₙ)NR¹⁰R¹¹ mit den oben genannten Bedeutungen, bedeutet, und
S_{cl} Wasserstoff ist oder eine Schutzgruppe ausgewählt aus einer Acyl-, Trityl-, Fluorenyhnethyloxycarbonyl- oder photolabilen Schutzgruppe, eine Dansyl- oder Allyloxycarbonylgruppe, insbesondere eine Benzoyl- oder 4, 4'-Dimethoxytrityl-(DMT-)gruppe,
oder der Formel (II) 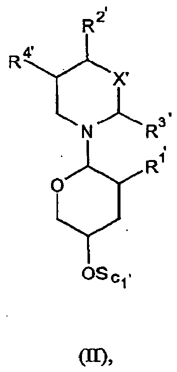 worin R^{1'} gleich OH oder Hal mit Hal gleich Br oder Cl, ein Rest ausgewählt aus 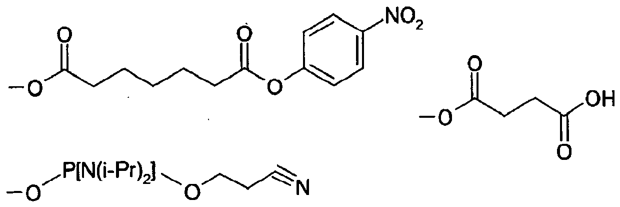 mit i-Pr gleich Isopropyl, oder -O-PH-(=O)(-O⁻), R^{2'},R^{3'} und R^{4'} unabhängig voneinander, gleich oder verschieden, jeweils =O, CₙH₂ₙ₊₁ oder OCₙH₂ₙ₋₁, eine ß-eliminierbare Gruppe der Formel - OCH₂CH₂R¹⁸ mit R¹⁸ gleich ein Cyano- oder p-Nitrophenylrest oder ein Fluorenylmethyloxycarbonyl-(Fmoc)-Rest, oder (CₙH₂ₙ)NR¹⁰R¹¹, wobei R¹⁰ R¹¹ unabhängig voneinander die oben genannte Bedeutung von R¹⁰ bzw. R¹¹ hat, und
X' =N-, =C(R^{16'})- oder -N(R^{17'})- bedeutet, wobei R^{16'} und R^{17'} unabhängig voneinander die oben genannte Bedeutung von R¹⁶ bzw. R¹⁷ haben, und S_{cl}, die oben genannte Bedeutung von S_{cl} hat.

2. 3'-Desoxypentopyranosyl-Nucleoside nach Anspruch 1, **dadurch gekennzeichnet, daß** das 3'-Desoxypentopyranosyl-Nucleosid ein 3'-Desoxyribo-, 3'-Desoxyarabino-, 3'-Desoxylyxo- und/oder 3'-Desoxyxylo-pyranosylNucleosid ist, vorzugsweise ein 3'-Desoxyribopyranosyl-Nucleosid.

3. 3'-Desoxypentopyranosyl-Nucleoside nach einem der Ansprüche 1 oder 2, **dadurch gekennzeichnet, daß** der 3'-Desoxypentopyranosyl -Teil D- oder L-konfiguriert ist.

4. 3'-Desoxypentopyranosyl-Nucleoside nach einem der Ansprüche 1-2, **dadurch gekennzeichnet, daß** das 3'-Desoxypentopyranosyl-Nucleosid ein 3'-Desoxypentopyranosyl-purin, -2,6-diaminopurin, -6-purinthiol, -adenin,-guanin, -isoguanin, -xanthin, -hypoxanthin, -thymin, -indol, -tryptamin, -N-phthaloyltryptamin, -coffein, -theobromin oder -theophyllinist.

5. 3'-Desoxypentopyranosyl-Nucleoside nach einem der Ansprüche 1-4, **dadurch gekennzeichnet, daß** R², R³ und/oder R⁴ ein 2-Phtalimidoethyl- oder Allyloxy-Rest oder ein Rest der Formel-N[C(O)R⁹]₂ bedeutet und/oder R^{2'}, R^{3'} und/oder R^{4'} ein 2-Phtalimidoethyl-Rest bedeutet.

6. 3'-Desoxypentopyranosyl-Nucleoside nach einem der Ansprüche 1-5, **dadurch gekennzeichnet, daß** das 3'-Desoxypentopyranosyl-Nucleosid ein [(2', 4'-Di-O-Benzoyl)-3'-desoxy-β-ribopyranosyl]-nucleosid, ein N-Benzoyl-2', 4'-di-O-benzoyl-3'-desoxy-ribopyranosyl-Nucleosid, ein N-Isobutyroyl-2', 4'-di-O-benzoyl-3'-desoxy-ribopyranosyl-Nucleosid, ein O⁶-(2-(4-Nitrophenyl)ethyl)-N²-isobutyroyl-2',4'-di-O-benzoyl-3'-desoxy-ribopyranosyl-Nucleosid, 3'-Desoxy-β-ribopyranosyl-Nueleosid, ein N-Benzoyl-, N-Isobutyroyl-, O⁶-(2-Cyanoethyl)- oder O⁶-(2-(4-Nitrophenyl)ethyl)-N²-isobutyroyl-3'-Desoxy-ß-ribopyranosyl-nucleosid, 4'-DMT-3'-desoxypentopyrauosyl-Nueleosid, vorzugsweise ein 4'-DMT-3'-desoxyribopyranosyl-Nucleosid, N-Benzoyl-4'-DMT-3'-desoxy-ribopyranosyl-Nucleosid, N-Isobutyroyl-4'-DMT3'-desoxyribopyranosl-Nucleosid, O⁶-(2-Cyanoethyl)-N²-isobutyroyl-4'-DMT-3'-desoxyribopyranosyl-Nucleosid, O⁶-(2-(4-Nitrophenyl)ethyl)-N²-isobutyroyl-4'-DMT-3'-desoxyribopyranosyl-Nucleosid, ein 3'-Desoxy-β-ribopyranosyl-N,N'-dibenzoyl-adenosin oder ein 3'-Desoxy-β-ribopyranosyl-N,N'-dibenzoyl-guanosin ist.

7. Verfahren zur Herstellung einer 3'-Desoxypentopyranosyl-Nucleinsäure, **dadurch gekennzeichnet, daß**
(a) in einem ersten Schritt ein geschütztes 3'-Desoxypentopyranosyl-Nucleosid gemäß einem der Ansprüche 1-6 an eine feste Phase gebunden wird und
(b) in einem zweiten Schritt das gemäß Schritt (a) an eine feste Phase gebundene 4'- geschützte 3'-Desoxypentopyranosylnukleosid um ein 2'-phosphityliertes 4'- geschütztes 3'-Desoxypentopyranosyl-Nucleosid verlängert wird, und
(c) Schritt (b) wiederholt wird.

8. Verfahren nach Anspruch 7 **dadurch gekennzeichnet, daß** in Schritt (a) und/oder Schritt (b) auch Pentofuranosyl-nucleoside eingebaut werden.

9. Verfahren nach Anspruch 7 oder 8, **dadurch gekennzeichnet, daß** als Kupplungsreagenz für die Verlängerung gemäß Schritt (b) Pyridinium-Hydrochlorid bei Einsatz von Phosphoramiditen und bei Einsatz von H-Phosphonaten Arylsulfonylchloride, Diphenylchlorophosphat, Pivaloylchlorid oder Adamantoylchlorid eingesetzt wird.

10. Verfahren nach einem der Ansprüche 7-9, **dadurch gekennzeichnet, daß** in einem weiteren Schritt (d) die Schutzgruppen und das gebildete Oligomer von der festen Phase abgespalten wird.

11. 3'-Desoxypentopyranosyl-Nucleinsäuren **dadurch gekennzeichnet, dass** sie aus 3'-Desoxypentopyranosyl-Nucleosiden gemäß einem der Ansprüche 1 bis 6
aufgebaut sind.

12. Verwendung von 3'-Desoxypentopyranosyl-Nucleosiden nach einem der Ansprüche 1 bis 6 zur Herstellung von 3'-Desoxypentopyranosyl-Nucleinsäuren.

13. Konjugat enthaltend eine 3'-Desoxypentopyranosyl-Nucleinsäure gemäß Anspruch 11 und ein Biomolekül ausgewählt aus der Gruppe der Peptide, Proteine oder Nucleinsäuren.

14. Konjugat nach Anspruch 13, **dadurch gekennzeichnet, daß** das Biomolekül ein Antikörper oder ein funktioneller Teil davon oder eine DNA und/oder RNA ist.

## Claims

1. 3'-deoxypentopyranosylnucleosides of the formula (I), 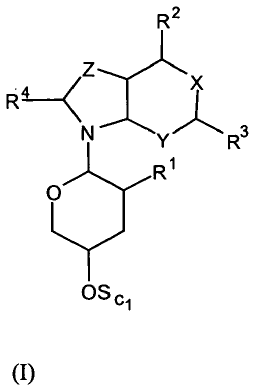 in which
R¹ is equal to OH, Hal where Hal is equal to Br or Cl, a radical selected from 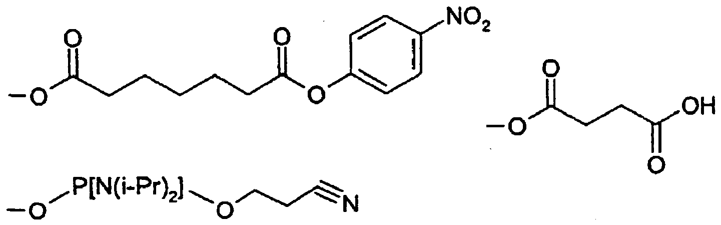 where i-Pr is equal to isopropyl, or -O-PH-(=O)(-O⁻),
R², R³ and R⁴ independently of one another, identically or differently, are in each case H, NR⁵R⁶, OR⁷, SR⁸, =O, CₙH₂₊₁ where n is an integer from 1-12, preferably 1-8, in particular 1-4, a β-eliminable group of the formula -OCH₂CH₂R¹⁸ where R¹⁸ is equal to a cyano or p-nitrophenyl radical or a fluorenylmethyloxycarbonyl (Fmoc) radical, or (CₙH₂ₙ)NR¹⁰R¹¹ where R¹⁰R¹¹ is equal to H, CₙH₂ₙ₊₁ or R¹⁰R¹¹ are bonded via a radical of the formula 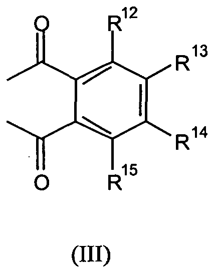 in which R¹², R¹³, R¹⁴ and R¹⁵ independently of one another, identically or differently, are in each case H, OR⁷, or CₙH₂ₙ₊₁, or CₙH₂ₙ₋₁, where n has the abovementioned meaning, and
R⁵, R⁶, R⁷ and R⁸ independently of one another, identically or differently, are in each case H, CₙH₂ₙ₊₁, or CₙH₂ₙ₋₁, where n has the abovementioned meaning, -C(O)R⁹ where R⁹ is equal to a linear or branched, optionally substituted alkyl or aryl radical, preferably a phenyl radical,
X, Y and Z independently of one another, identically or differently, are in each case =N-, =C(R¹⁶)- or -N(R¹⁷)- where R¹⁶ and R¹⁷ independently of one another, identically or differently, are in each case H or CₙH₂ₙ₊₁ or (CₙH₂ₙ)NR¹⁰R¹¹ with the abovementioned meanings, and
S_{cl} is hydrogen or a protective group selected from an acyl, trityl, fluorenylmethyloxycarbonyl, or a photolabile protective group, a dansyl or allyloxycarbonyl, particularly a benzoyl or 4, 4'-dimethoxytrityl (DMT) group,
or of the formula (II) 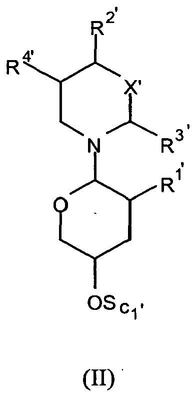 in which R^{1'} is equal to OH, Hal where Hal is equal to Br or Cl, a radical selected from 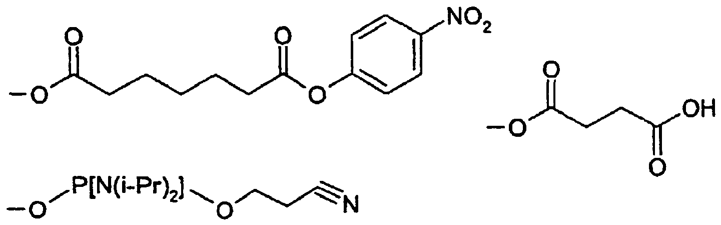 where i-Pr is equal to isopropyl, or -O-PH-(=O)(-O⁻),
R^{2'},R^{3'} and R^{4'} independently of one another, identically or differently, are in each case =O, CₙH₂ₙ₊₁ or OCₙH₂ₙ₋₁, a β-eliminable group of the formula -OCH₂CH₂R¹⁸ where R¹⁸ is equal to a cyano or p-nitrophenyl radical or a fluorenylmethyloxy-carbonyl (Fmoc) radical or (CₙH₂ₙ)NR¹⁰R¹¹, where R¹⁰, R¹¹, independently of one another has the abovementioned meaning of R¹⁰ or R¹¹, and
X' is equal to =N-, =C(R^{16'})- or -N(R^{17'})-, where R^{16'} and R^{17'} independently of one another have the abovementioned meaning of R¹⁶ and R¹⁷, and S_{cl}, has the abovementioned meaning of S_{cl}.

2. 3'-Deoxypentopyranosylnucleoside according to Claim 1, **characterized in that** the 3'-deoxypentopyranosylnucleoside is a 3'-deoxyribo-, 3'-deoxyarabino-, 3'-deoxylyxo- and/or 3'-deoxyxylopyranosylnucleoside, preferably a 3'-deoxyribopyranosylnucleoside.

3. 3'-Deoxypentopyranosylnucleoside according to one of Claims 1 or 2, **characterized in that** the 3'-deoxypentopyranosyl moiety is of D or L configuration.

4. 3'-Deoxypentopyranosylnucleoside according to one of Claims 1-2, **characterized in that** the 3'-deoxypentopyranosylnucleoside is a 3'-deoxypentopyranosylpurine, -2,6-diaminopurine, -6-purinethiol, -adenine, -guanine, -isoguanine, -xanthine, -hypoxanthine, -thymine, -indole, -tryptamine, -N-phthaloyltryptamine, -caffeine, -theobromine or -theophylline.

5. 3'-Deoxypentopyranosylnucleoside according to one of Claims 1-4, **characterized in that** R², R³ and/or R⁴ is a phthalimidoethyl or allyloxy radical or a radical of the formula -N[C(O)R⁹]₂ and/or R^{2'}, R^{3'} and/or R^{4'} is a phthalimidoethyl.

6. 3'-Deoxypentopyranosylnucleoside according to one of Claims 1-5, **characterized in that** the 3'-deoxypentopyranosylnucleoside is a [(2', 4'-di-O-benzoyl)-3'-deoxy-β-ribopyranosyl]nucleoside, an N-benzoyl-2', 4'-di-O-benzoyl-3'-deoxyribopyranosylnucleoside, an N-isobutyroyl-2', 4'-di-O-benzoyl-3'-deoxy-ribopyranosylnucleoside, an O⁶-(2-(4-nitrophenyl)ethyl)-N²-isobutyroyl-2',4'-di-O-benzoyl-3'-deoxyribopyranosylnucleoside, 3'-deoxy-β-ribopyranosylnucleoside, an N-benzoyl-, N-isobutyroyl,- O⁶-(2-cyanoethyl)- or O⁶-(2-(4-nitrophenyl)ethyl)-N²-isobutyroyl-3'-deoxy-β-ribopyranosylnucleoside, 4'-DMT-3'-deoxypentopyranosyl-nucleoside, preferably a 4'-DMT-3'-deoxyribopyranosylnucleoside, N-benzoyl-4'-DMT-3'-deoxy-ribopyranosylnucleoside, N-isobutyroyl-4'-DMT3'-deoxyribopyranosylnucleoside, O⁶-(2-cyanoethyl)-N²-isobutyroyl-4'-DMT-3'-deoxyribopyranosylnucleoside, O⁶-(2-(4-nitrophenyl)ethyl)-N²-isobutyroyl-4'-DMT-3'-deoxyribopyranosylnucleoside, a 3'-deoxy-β-ribopyranosyl-N,N'-dibenzoyladenosine or a 3'-deoxy-β-ribopyranosyl-N,N'-dibenzoylguanosine.

7. Process for the preparation of a 3'-deoxypentopyranosylnucleic acid, **characterized in that**
(a) in a first step a protected 3'-deoxypentopyranosylnucleoside according to one of Claims 1-6 is bonded to a solid phase and
(b) in a second step the 4'-protected 3'-deoxypentopyranosylnucleoside bonded to a solid phase according to step (a) is lengthened by a 2'-phosphitylated 4'-protected 3'-deoxypentopyranosylnucleoside, and
(c) step (b) is repeated.

8. Process according to Claim 7, **characterized in that** in step (a) and/or step (b) pentofuranosylnucleosides are also incorporated.

9. Process according to Claim 7 or 8, **characterized in that** the coupling reagent employed for the lengthening according to step (b) is pyridinium hydrochloride when using phosphoramidites and, when using H-phosphonates, arylsulphonyl chlorides, diphenyl chlorophosphate, pivaloyl chloride or adamantoyl chloride.

10. Process according to one of Claims 7-9, **characterized in that** in a further step (d) the protective groups and the oligomer formed are cleaved from the solid phase.

11. 3'-Deoxypentopyranosylnucleic acids, **characterized in that** the 3'-Deoxypentopyranosylnucleic acids are composed of 3'-Deoxypentopyranosyl nucleosides according to one of the Claims 1 to 6.

12. Use of a 3'-deoxypentopyranosyl nucleosides according to one of Claims 1-6 for the preparation of 3'-deoxypentopyranosylnucleic acids.

13. Conjugate comprising a 3'-deoxypentopyranosylnucleic acid according to Claim 11 and a biomolecule selected from peptides, proteins or nucleic acids.

14. Conjugate according to Claim 13, **characterized in that** the biomolecule is an antibody or a functional moiety thereof or a DNA and/or RNA.

## Revendications

1. Blocs 3'-désoxypentopyranosylnucléosides de formule (I), 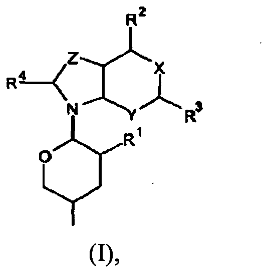 dans laquelle
R¹ est OH ; Hal, où Hal est Br ou Cl ; un reste choisi parmi 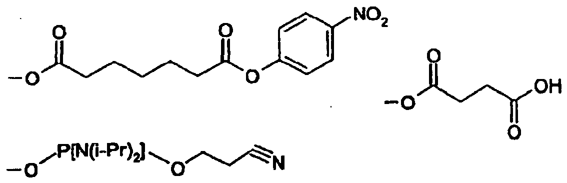 où i-Pr est l'isopropyle, ou -O-PH-(=O)(-O),
R², R³ et R⁴, identiques ou différents, sont chacun indépendamment des autres H ; NR⁵R⁶; OR⁷; SR⁸ ; =O ; CₙH₂ₙ₊₁, où n est un nombre entier valant de 1 à 12, de préférence de 1 à 8 et en particulier de 1 à 4 ; un groupe β-éliminable de formule -OCH₂CH₂R¹⁸, où R¹⁸ est un reste cyano ou p-nitrophényle ou un reste fluorénylméthyloxycarbonyle (Fmoc) ; ou (CₙH₂ₙ)NR¹⁰R¹¹, où R¹⁰R¹¹ est H, CₙH₂ₙ₊₁ ou bien
R¹⁰R¹¹ rattaché par l'intermédiaire d'un reste de formule 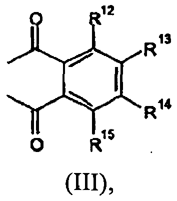 dans laquelle
R¹², R¹³, R¹⁴ et R¹⁵, identiques ou différents, sont chacun indépendamment des autres H, OR⁷ ou bien CₙH₂ₙ₊₁ ou CₙH₂ₙ₋₁, où n a la valeur indiquée plus haut, et
R⁵, R⁶, R⁷ et R⁸, identiques ou différents, sont chacun indépendamment des autres H, CₙH₂ₙ₊₁ ou CₙH₂ₙ₋₁, où n a la valeur indiquée plus haut, -C(O)R⁹, où R⁹ est un reste alkyle ou aryle linéaire ou ramifié, le cas échéant substitué, de préférence un phényle,
X, Y et Z, identiques ou différents, sont chacun indépendamment des autres =N-, =C(R¹⁶)- ou -N(R¹⁷)-, où R¹⁶ et R¹⁷, identiques ou différents, sont chacun indépendamment des autres H ou bien CₙH₂ₙ₊₁ ou (CₙH₂ₙ)NR¹⁰R¹¹, où les symboles ont les significations indiquées plus haut, et
S_{cl} est l'hydrogène ou un groupe protecteur choisi parmi un acyle, un trityle, un fluorénylméthyloxycarbonyle ou un groupe protecteur photolabile, un groupe dansyle ou allyloxycarbonyle, en particulier un groupe benzoyle ou 4,4'-diméthoxytrityle (DMT),
ou un groupe de formule (II) 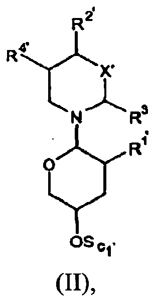 dans laquelle
R^{1'} est OH ; Hal, où Hal est Br ou Cl ; un reste choisi parmi 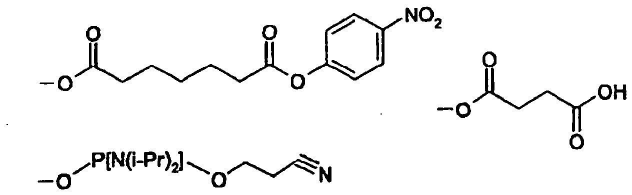 où i-Pr est l'isopropyle ; ou -O-PH-(=O)(-O'),
R^{2'}, R^{3'} et R^{4'}, identiques ou différents, sont chacun indépendamment des autres =O, CₙH₂ₙ₊₁ ou OCₙH₂ₙ₋₁, un groupe β-éliminable de formule -OCH₂CH₂R¹⁸, où R¹⁸ est un reste cyano ou p-nitrophényle ou un reste fluorénylméthyloxycarbonyle (Fmoc), ou (CₙH₂ₙ)NR¹⁰R¹¹, où les restes dans R¹⁰R¹¹ ont indépendamment l'un de l'autre la signification indiquée plus haut pour R¹⁰ ou R¹¹, et
X' est =N-, =C(R¹⁶)- ou -N(R^{17'})-, où R^{16'} et R^{17'} ont indépendamment l'un de l'autre la signification indiquée plus haut pour R¹⁶ ou R¹⁷ et S_{cl'} a la signification indiquée plus haut pour S_{cl}.

2. 3'-Désoxypentopyranosylnucléosides selon la revendication 1, **caractérisés en ce que** le 3'-désoxypentopyranosylnucléoside est un 3'-désoxyribo-, 3'-désoxy-arabino-, 3'-désoxyxylo- et/ou 3'-désoxyxylopyranosylnucléoside, de préférence un 3'-désoxyribopyranosylnucléoside.

3. 3'-Désoxypentopyranosylnucléosides selon l'une des revendications 1 ou 2, **caractérisés en ce que** la partie 3'-désoxypentopyranosyle présente une configuration D ou L.

4. 3'-Désoxypentopyranosylnucléosides selon l'une des revendications 1 à 2, **caractérisés en ce que** le 3'-désoxypentopyranosylnucléoside est une 3'-désoxypento-pyranosylpurine, -2,6-diaminopurine, -6-purinethiol, -adénine, -guanine, -isoguanine, -xanthine, -hypoxanthine, -thymine, -indole, -tryptamine, -N-phtaloyltryptamine, -caféine, -théobromine ou -théophylline.

5. 3'-Désoxypentopyranosylnucléosides selon l'une des revendications 1 à 4, **caractérisés en ce que** R², R³ et/ou R⁴ représentent un reste 2-phtalimidoéthyle ou allyloxy ou un reste de formule N[C(O)R⁹]₂ et/ou R^{2'}, R^{3'} et/ou R^{4'} représentent un reste 2-phtalimidoéthyle.

6. 3'-Désoxypentopyranosylnucléosides selon l'une des revendications 1 à 5, **caractérisés en ce que** le 3'-désoxypentopyranosylnucléoside est un [(2',4'-di-O-benzoyl)-3'-désoxy-β-ribopyranosyl]nucléoside, un N-benzoyl-2',4'-di-O-benzoyl-3'-désoxyribopyranosylnucléoside, un N-isobutyroyl-2',4'-di-O-benzoyl-3'-désoxyribopyranosylnucléoside, un O⁶-(2-(4-nitrophényl)éthyl)-N²-isobutyroyl-2',4'-di-O-benzoyl-3'-désoxyribopyranosylnucléoside, un 3'-désoxy-β-ribopyranosylnucléoside, un N-benzoyl-, N-isobutyroyl-, O⁶-(2-cyanoéthyl)- ou O⁶-(2-(4-nitrophényl)éthyl)-N²-isobutyroyl-3'-désoxy-β-ribopyranosylnucléoside, un 4'-DMT-3'-désoxypentopyranosylnucléoside, de préférence un 4'-DMT-3'désoxyribopyranosylnucléoside, un N-benzoyl-4'-DMT-3'-désoxyribopyranosylnucléoside, un N-isobutyroyl-4'-DMT-3'-désoxyribopyranosylnucléoside, un O⁶-(2-cyanoéthyl)-N²-isobutyroyl-4'-DMT-3'-désoxyribopyranosylnucléoside, un O⁶-(2-(4-nitrophényl)éthyl)-N²-isobutyroyl-4'-DMT-3'-désoxyribopyranosylnucléoside, une 3'-désoxy-β-ribopyranosyl-N,N'-dibenzoyladénosine ou une 3'-désoxy-β-ribopyranosyl-N,N'-dibenzoylguanosine.

7. Procédé de préparation d'un acide 3'-désoxypentopyranosylnucléique, **caractérisé en ce que**
(a) dans une première étape, un 3'-désoxypentopyranosylnucléoside protégé selon l'une des revendications 1 à 6 est lié à une phase solide, et
(b) dans une deuxième étape, le 3'-désoxypentopyranosylnucléoside protégé en 4' lié à une phase solide à l'étape (a) est allongé d'un 3'-désoxypentopyranosylnucléoside phosphitylé en 2' et protégé en 4', et
(c) l'étape (b) est répétée.

8. Procédé selon la revendication 7, **caractérisé en ce que** des pentofuranosyl-nucléosides sont également incorporés à l'étape (a) et/ou à l'étape (b).

9. Procédé selon la revendication 7 ou la revendication 8, **caractérisé en ce que** le réactif de couplage utilisé pour l'allongement de l'étape (b) est du chlorhydrate de pyridinium en cas d'utilisation de phosphoramidites et des chlorures d'arylsulfonyles, du chlorophosphate de diphényle, du chlorure de pivaloyle ou du chlorure d'adamantoyle en cas d'utilisation d'hydrogénophosphonates.

10. Procédé selon l'une des revendications 7 à 9, **caractérisés en ce que** les groupes protecteurs et l'oligomère formé sont dissociés de la phase solide dans une étape (d) supplémentaire.

11. Acides 3'-désoxypentopyranosylnucléiques, **caractérisés en ce qu'**ils sont constitués de 3'-désoxypentopyranosylnucléosides selon l'une des revendications 1 à 6.

12. Utilisation de 3'-désoxypentopyranosylnucléosides selon l'une des revendications 1 à 6 pour produire des acides 3'-désoxypentopyranosylnucléiques.

13. Conjugué contenant un acide 3'-désoxypentopyranosylnucléique selon la revendication 11 et une biomolécule choisie dans le groupe des peptides, des protéines ou des acides nucléiques.

14. Conjugué selon la revendication 13, **caractérisé en ce que** la biomolécule est un anticorps ou un fragment d'anticorps fonctionnel ou un ADN et/ou de l'ARN.
